# EUROPEAN PATENT APPLICATION

(11) **EP 3 130 589 A1**
(43) Date of publication of application: **15.02.2017**
(21) Application number: 15002405.7
(22) Date of filing: 13.08.2015
(51) Int. Cl.: C07D 401/14, C07D 405/14, C07D 413/14, C07D 401/12, C07D 417/14, A61K 31/4439, A61K 31/428, A61K 31/351, A61P 29/00

(54) **HETEROCYCLIC AZA COMPOUNDS**

(71) Applicant: Grünenthal GmbH, 52078 Aachen (DE)
(72) Inventor: Frank-Foltyn, Robert, 34323 Beiseförth (DE); Chistoph, Thomas, 52080 Aachen (DE); Lesch, Bernhard, 52134 Herzogenrath (DE); Lee, Jewoo, Seoul 151-742 (KR)

(57) **Abstract**

The invention relates to heterocyclic aza compounds as vanilloid receptor ligands, to pharmaceutical compositions containing these compounds and also to these compounds for use in the treatment and/or prophylaxis of pain and further diseases and/or disorders.

## Description

The invention relates to heterocyclic aza compounds as vanilloid receptor ligands, to pharmaceutical compositions containing these compounds and also to these compounds for use in the treatment and/or prophylaxis of pain and further diseases and/or disorders.

The treatment of pain, in particular of neuropathic pain, is very important in medicine. There is a worldwide demand for effective pain therapies. The urgent need for action for a patient-focused and target-oriented treatment of chronic and non-chronic states of pain, this being understood to mean the successful and satisfactory treatment of pain for the patient, is also documented in the large number of scientific studies which have recently appeared in the field of applied analgesics or basic research on nociception.

The subtype 1 vanilloid receptor (VR1/TRPV1), which is often also referred to as the capsaicin receptor, is a suitable starting point for the treatment of pain, in particular of pain selected from the group consisting of acute pain, chronic pain, neuropathic pain and visceral pain. This receptor is stimulated inter alia by vanilloids such as capsaicin, heat and protons and plays a central role in the formation of pain. In addition, it is important for a large number of further physiological and patho-physiological processes and is a suitable target for the therapy of a large number of further disorders such as, for example, migraine, depression, neurodegenerative diseases, cognitive disorders, states of anxiety, epilepsy, coughs, diarrhoea, pruritus, inflammations, disorders of the cardiovascular system, eating disorders, medication dependency, misuse of medication and urinary incontinence.

There is a demand for further compounds having comparable or better properties, not only with regard to affinity to vanilloid receptors 1 (VR1/TRPV1 receptors) per se (*potency, efficacy*).

Thus, it may be advantageous to improve the metabolic stability, the solubility in aqueous media or the permeability of the compounds. These factors can have a beneficial effect on oral bioavailability or can alter the PK/PD (pharmacokinetic/pharmacodynamic) profile; this can lead to a more beneficial period of effectiveness, for example.

It was therefore an object of the invention to provide novel compounds, preferably having advantages over the prior-art compounds. The compounds should be suitable in particular as pharmacologically active ingredients in pharmaceutical compositions, preferably in pharmaceutical compositions for the treatment and/or prophylaxis of disorders or diseases which are at least partially mediated by vanilloid receptors 1 (VR1/TRPV1 receptors).

This object is achieved by the subject matter of the claims and the subject-matter as described herein.

It has surprisingly been found that the compounds of general formula (I), as given below, display outstanding affinity to the subtype 1 vanilloid receptor (VR1/TRPV1 receptor) and are therefore particularly suitable for the prophylaxis and/or treatment of disorders or diseases which are at least partially mediated by vanilloid receptors 1 (VR1/TRPV1).

The present invention relates to a compound of formula (I) 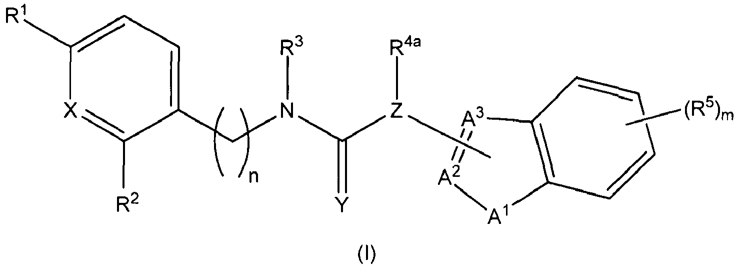 wherein
n represents 1, 2, 3 or 4;
m represents 0, 1, 2, 3 or 4;
X represents N or CH;
Y represents O, S, or N-CN;
Z represents N or CR^{4b};
A¹ represents S, O or NR⁶;
A² represents N or CR⁷;
A³ represents N or CR⁷;
with the proviso that one of R⁶ and R⁷ represents a bond to Z;
- R¹: represents a C₁₋₄ aliphatic residue, a C₃₋₆ cycloaliphatic residue or a 3 to 6 membered heterocycloaliphatic residue, in each case unsubstituted or mono- or polysubstituted;
- R²: represents an unsubstituted or mono- or polysubstituted C₁₋₁₀ aliphatic residue,
a C₃₋₁₀-cycloaliphatic residue, a 3 to 10-membered heterocycloaliphatic residue, an aryl or a heteroaryl, in each case unsubstituted or mono- or polysubstituted and in each case optionally bridged via an unsubstituted or mono- or polysubstituted C₁₋₈ aliphatic group;
O-C₁₋₁₀ aliphatic residue, O-C₃₋₁₀ cycloaliphatic residue, 0-(3 to 10-membered heterocycloaliphatic residue), O-aryl or O-heteroaryl, in each case unsubstituted or mono- or polysubstituted;
(O-(CH₂)₁₋₃)₀₋₃-O-(CH₂)₀₋₃-CH₃, optionally bridged via an unsubstituted or mono- or polysubstituted C₁₋₈ aliphatic group;
S-C₁₋₁₀ aliphatic residue, S-C₃₋₁₀ cycloaliphatic residue, S-(3 to 10-membered heterocycloaliphatic residue), S-aryl or S-heteroaryl, in each case unsubstituted or mono- or polysubstituted;
NH₂;
N(H)-C₁₋₁₀ aliphatic residue, N(H)-C₃₋₁₀ cycloaliphatic residue, N(H)-(3 to 10-membered heterocycloaliphatic residue), N(H)-aryl or N(H)-heteroaryl, in each case unsubstituted or mono-or polysubstituted;
N(C₁₋₄ aliphatic residue)-C₁₋₁₀ aliphatic residue, N(C₁₋₄ aliphatic residue)-C₃₋₁₀ cycloaliphatic residue, N(C₁₋₄ aliphatic residue)-(3 to 10-membered heterocycloaliphatic residue), N(C₁₋₄ aliphatic residue)-aryl or N(C₁₋₄ aliphatic residue)-heteroaryl, in each case unsubstituted or mono- or polysubstituted;
- R³: represents H or an unsubstituted or mono- or polysubstituted C₁₋₄ aliphatic residue;
- R^{4a}: represents H; a C₁₋₄ aliphatic residue, a C₃₋₆ cycloaliphatic residue or aryl, in each case unsubstituted or mono- or polysubstituted;
- R^{4b}: represents H; or an unsubstituted, mono- or polysubstituted C₁₋₄ aliphatic residue;
- R⁵: each independently of one another represents F; Cl; Br; I; CN; CH₃; C₂₋₄-alkyl; CF₃; CF₂H; CFH₂; CF₂Cl; CFCl₂; C(=O)-H; C(=O)-(C₁₋₄-alkyl); C(=O)-OH;C(=O)-O-(C₁₋₄-alkyl); C(=O)-NH₂; C(=O)-N(H)(C₁₋₄-alkyl); C(=O)-N(C₁₋₄-alkyl)₂; OH; OCH₃; O-C₂₋₄-alkyl; OCF₃; OCF₂H; OCFH₂; OCF₂Cl; OCFCl₂; O-S(=O)₂-OH; O-S(=O)₂-O-C₁₋₄-alkyl; O-S(=O)₂-NH₂; O-S(=O)₂-N(H)(C₁₋₄-alkyl); O-S(=O)₂-N(C₁₋₄-alkyl)₂; NH₂; NO₂; N(H)(C₁₋₄-alkyl); N(C₁₋₄-alkyl)₂; NH-C(=O)-NH₂; NH-C(=O)-N(H)(C₁₋₄-alkyl); NH-C(=O)-N(C₁₋₄-alkyl)₂; NH-S(=O)₂-OH; NH-S(=O)₂-O-C₁₋₄-alkyl; NH-S(=O)₂-NH₂; NH-S(=O)₂-N(H)(C₁₋₄-alkyl); NH-S(=O)₂-N(C₁₋₄-alkyl)₂; SH; SCH₃; S-C₂₋₄-alkyl; SCF₃; SCF₂H; SCFH₂; SCF₂Cl; SCFCl₂; S(=O)₂-OH; S(=O)₂-O-C₁₋₄-alkyl; S(=O)₂-NH₂; S(=O)₂-N(H)(C₁₋₄-alkyl); or S(=O)₂-N(C₁₋₄-alkyl)₂;
- R⁶: represents H; a C₁₋₄ aliphatic residue, a C₃₋₆-cycloaliphatic residue or a 3 to 6-membered heterocycloaliphatic residue, in each case unsubstituted or mono- or polysubstituted;
- R⁷: represents bond; H; F; Cl; Br; I; CN; CH₃; C₂₋₄-alkyl; CF₃; CF₂H; CFH₂; CF₂Cl; CFCl₂; C(=O)-H; C(=O)-(C₁₋₄-alkyl); C(=O)-OH;C(=O)-O-(C₁₋₄-alkyl); C(=O)-NH₂; C(=O)-N(H)(C₁₋₄-alkyl); C(=O)-N(C₁₋₄-alkyl)₂; OH; OCH₃; O-C₂₋₄-alkyl; OCF₃; OCF₂H; OCFH₂; OCF₂Cl; OCFCl₂; O-S(=O)₂-OH; O-S(=O)₂-O-C₁₋₄-alkyl; O-S(=O)₂-NH₂; O-S(=O)₂-N(H)(C₁₋₄-alkyl); O-S(=O)₂-N(C₁₋₄-alkyl)₂; NH₂; NO₂; N(H)(C₁₋₄-alkyl); N(C₁₋₄-alkyl)₂; NH-C(=O)-NH₂; NH-C(=O)-N(H)(C₁₋₄-alkyl); NH-C(=O)-N(C₁₋₄-alkyl)₂; NH-S(=O)₂-OH; NH-S(=O)₂-O-C₁₋₄-alkyl; NH-S(=O)₂-NH₂; NH-S(=O)₂-N(H)(C₁₋₄-alkyl); NH-S(=O)₂-N(C₁₋₄-alkyl)₂; SH; SCH₃; S-C₂₋₄-alkyl; SCF₃; SCF₂H; SCFH₂; SCF₂Cl; SCFCl₂; S(=O)₂-OH; S(=O)₂-O-C₁₋₄-alkyl; S(=O)₂-NH₂; S(=O)₂-N(H)(C₁₋₄-alkyl); or S(=O)₂-N(C₁₋₄-alkyl)₂;
in which an "aliphatic group" and an "aliphatic residue" can in each case, independently of one another, be branched or unbranched, saturated or unsaturated;
in which a "cycloaliphatic residue" and a "heterocycloaliphatic residue" can in each case, independently of one another, be saturated or unsaturated;
in which "mono- or polysubstituted" with respect to an "aliphatic group", an "aliphatic residue", a "cycloaliphatic residue" and a "heterocycloaliphatic residue" relates in each case independently of one another, with respect to the corresponding residues or groups, to the substitution of one or more hydrogen atoms each independently of one another by at least one substituent selected from the group consisting of F; Cl; Br; I; CN; CH₃; C₂₋₄-alkyl; CF₃; CF₂H; CFH₂; CF₂Cl; CFCl₂; C(=O)-H; C(=O)-(C₁₋₄-alkyl); C(=O)-OH;C(=O)-O-(C₁₋₄-alkyl); C(=O)-NH₂; C(=O)-N(H)(C₁₋₄-alkyl); C(=O)-N(C₁₋₄-alkyl)₂; OH; OCH₃; O-C₂₋₄-alkyl; OCF₃; OCF₂H; OCFH₂; OCF₂Cl; OCFCl₂; O-S(=O)₂-OH; O-S(=O)₂-O-C₁₋₄-alkyl; O-S(=O)₂-NH₂; O-S(=O)₂-N(H)(C₁₋₄-alkyl); O-S(=O)₂-N(C₁₋₄-alkyl)₂; NH₂; NO₂; N(H)(C₁₋₄-alkyl); N(C₁₋₄-alkyl)₂; NH-C(=O)-NH₂; NH-C(=O)-N(H)(C₁₋₄-alkyl); NH-C(=O)-N(C₁₋₄-alkyl)₂; NH-S(=O)₂-OH; NH-S(=O)₂-O-C₁₋₄-alkyl; NH-S(=O)₂-NH₂-, NH-S(=O)₂-N(H)(C₁₋₄-alkyl); NH-S(=O)₂-N(C₁₋₄-alkyl)₂; SH; SCH₃; S-C₂₋₄-alkyl; SCF₃; SCF₂H; SCFH₂; SCF₂Cl; SCFCl₂; S(=O)₂-OH; S(=O)₂-O-C₁₋₄-alkyl; S(=O)₂-NH₂; S(=O)₂-N(H)(C₁₋₄-alkyl); or S(=O)₂-N(C₁₋₄-alkyl)₂;
in which "mono- or polysubstituted" with respect to "aryl" and a "heteroaryl" relates, with respect to the corresponding residues, in each case independently of one another, to the substitution of one or more hydrogen atoms each independently of one another by at least one substituent selected from the group consisting of F; Cl; Br; I; CN; CH₃; C₂₋₄-alkyl; CF₃; CF₂H; CFH₂; CF₂Cl; CFCl₂; C(=O)-H; C(=O)-(C₁₋₄-alkyl); C(=O)-OH;C(=O)-O-(C₁₋₄-alkyl); C(=O)-NH₂; C(=O)-N(H)(C₁₋₄-alkyl); C(=O)-N(C₁₋₄-alkyl)₂; OH; OCH₃; O-C₂₋₄-alkyl; OCF₃; OCF₂H; OCFH₂; OCF₂Cl; OCFCl₂; O-S(=O)₂-OH; O-S(=O)₂-O-C₁₋₄-alkyl; O-S(=O)₂-NH₂; O-S(=O)₂-N(H)(C₁₋₄-alkyl); O-S(=O)₂-N(C₁₋₄-alkyl)₂; NH₂; NO₂; N(H)(C₁₋₄-alkyl); N(C₁₋₄-alkyl)₂; NH-C(=O)-NH₂; NH-C(=O)-N(H)(C₁₋₄-alkyl); NH-C(=O)-N(C₁₋₄-alkyl)₂; NH-S(=O)₂-OH; NH-S(=O)₂-O-C₁₋₄-alkyl; NH-S(=O)₂-NH₂; NH-S(=O)₂-N(H)(C₁₋₄-alkyl); NH-S(=O)₂-N(C₁₋₄-alkyl)₂; SH; SCH₃; S-C₂₋₄-alkyl; SCF₃; SCF₂H; SCFH₂; SCF₂Cl; SCFCl₂; S(=O)₂-OH; S(=O)₂-O-C₁₋₄-alkyl; S(=O)₂-NH₂; S(=O)₂-N(H)(C₁₋₄-alkyl); or S(=O)₂-N(C₁₋₄-alkyl)₂;
optionally in the form of a single stereoisomer or a mixture of stereoisomers, in the form of the free compound and/or a physiologically acceptable salt thereof.

The term "single stereoisomer" comprises in the sense of this invention an individual enantiomer or diastereomer. The term "mixture of stereoisomers" comprises in the sense of this invention the racemate and mixtures of enantiomers and/or diastereomers in any mixing ratio.

The term "physiologically acceptable salt" comprises in the sense of this invention a salt of at least one compound according to the present invention and at least one physiologically acceptable acid or base.

The terms "C₁₋₁₀ aliphatic residue", "C₁₋₈ aliphatic residue", and "C₁₋₄ aliphatic residue" comprise in the sense of this invention acyclic saturated or unsaturated aliphatic hydrocarbon residues, which can be branched or unbranched and also unsubstituted or mono- or polysubstituted, which contain 1 to 10, or 1 to 8, or 1 to 4 carbon atoms. Preferably, aliphatic residues are selected from the group consisting of alkanyl (alkyl) and alkenyl residues, more preferred are alkanyl (alkyl) residues. Preferred alkanyl residues are selected from the group consisting of methyl, ethyl, n-propyl, 2-propyl, n-butyl, isobutyl, sec.-butyl, tert.-butyl, n-pentyl, isopentyl, neopentyl, n-hexyl, n-heptyl, n-octyl, n-nonyl and n-decyl.

The terms "C₃₋₆ cycloaliphatic residue" and "C₃₋₁₀ cycloaliphatic residue" mean for the purposes of this invention cyclic aliphatic hydrocarbons containing 3, 4, 5 or 6 carbon atoms and 3, 4, 5, 6, 7, 8, 9 or 10 carbon atoms, respectively, wherein the hydrocarbons in each case can be saturated or unsaturated (but not aromatic), unsubstituted or mono- or polysubstituted. The cycloaliphatic residues can be bound to the respective superordinate general structure via any desired and possible ring member of the cycloaliphatic residue. Preferred cycloaliphatic residues are selected from the group consisting of cyclopropyl, cyclobutyl, cyclopentyl, cyclohexyl, cycloheptyl, cyclooctyl, cyclononyl, cyclodecyl, adamantyl, 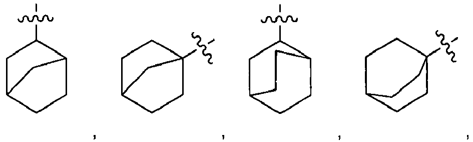 cyclopentenyl, cyclohexenyl, cycloheptenyl and cyclooctenyl. Particularly preferred cycloaliphatic residues are C₅₋₆ cycloaliphatic residues such as cyclopentyl, cyclohexyl, cyclopentenyl and cyclohexenyl.

The terms "3-6-membered heterocycloaliphatic residue", and "3-10-membered heterocycloaliphatic residue" mean for the purposes of this invention heterocycloaliphatic saturated or unsaturated (but not aromatic) residues having 3-6, i.e. 3, 4, 5 or 6 ring members, and 3-10, i.e. 3, 4, 5, 6, 7, 8, 9 or 10 ring members, respectively, in which in each case at least one, if appropriate also two or three carbon atoms are replaced by a heteroatom or a heteroatom group each selected independently of one another from the group consisting of O, S, S(=O)₂, N, NH and N(C₁₋₈ alkyl) such as N(CH₃); more preferably O, S, N, NH and N(C₁₋₈ alkyl) such as N(CH₃), wherein the ring members can be unsubstituted or mono- or polysubstituted. The heterocycloaliphatic residue can be bound to the superordinate general structure via any desired and possible ring member of the heterocycloaliphatic residue if not indicated otherwise. Preferred heterocycloaliphatic residues are selected from the group consisting of azetidinyl, aziridinyl, azepanyl, azocanyl, diazepanyl, dithiolanyl, dihydroquinolinyl, dihydropyrrolyl, dioxanyl, dioxolanyl, dioxepanyl, dihydroindenyl, dihydropyridinyl, dihydrofuranyl, dihydroisoquinolinyl, dihydroindolinyl, dihydroisoindolyl, imidazolidinyl, isoxazolidinyl, morpholinyl, oxiranyl, oxetanyl, oxazepanyl, pyrrolidinyl, piperazinyl, 4-methylpiperazinyl, piperidinyl, pyrazolidinyl, pyranyl, tetrahydropyrrolyl, tetrahydropyranyl, tetrahydro-2H-pyran-4-yl, tetrahydroquinolinyl, tetrahydroisoquinolinyl, tetrahydroindolinyl, tetrahydrofuranyl, tetrahydropyridinyl, tetrahydrothiophenyl, tetrahydropyridoindolyl, tetrahydronaphthyl, tetrahydrocarbolinyl, tetrahydroisoxazololyl, tetrahydropyridinyl, thiazolidinyl and thiomorpholinyl.

The term "aryl" means for the purpose of this invention aromatic hydrocarbons having 6 to 14, i.e. 6, 7, 8, 9, 10, 11, 12, 13 or 14 ring members, preferably having 6 to 10, i.e. 6, 7, 8, 9 or 10 ring members, including phenyls and naphthyls. Each aryl residue can be unsubstituted or mono- or polysubstituted, wherein the aryl substituents can be the same or different and in any desired and possible position of the aryl. The aryl can be bound to the superordinate general structure via any desired and possible ring member of the aryl residue. Preferably, aryl is selected from the group consisting of phenyl, 1-naphthyl, 2-naphthyl and anthracenyl, each of which can be respectively unsubstituted or mono- or polysubstituted. A particularly preferred aryl is phenyl, unsubstituted or mono- or polysubstituted.

The term "heteroaryl" for the purpose of this invention represents a 5 or 6-membered cyclic aromatic residue containing at least 1, if appropriate also 2, 3, 4 or 5 heteroatoms, wherein the heteroatoms are each selected independently of one another from the group S, N and O and the heteroaryl residue can be unsubstituted or mono- or polysubstituted. In the case of substitution on the heteroaryl, the substituents can be the same or different and be in any desired and possible position of the heteroaryl. The binding to the superordinate general structure can be carried out via any desired and possible ring member of the heteroaryl residue if not indicated otherwise. The heteroaryl can also be part of a bi- or polycyclic system having up to 14 ring members, wherein the ring system can be formed with saturated, (partially) unsaturated, (hetero)cycloaliphatic or aromatic or heteroaromatic rings, i.e. with a cycloaliphatic, heterocycloaliphatic, aryl or heteroaryl residue, which can in turn be unsubstituted or mono- or polysubstituted. It is preferable for the heteroaryl residue to be selected from the group consisting of benzofuranyl, benzoimidazolyl, benzothienyl, benzothiadiazolyl, benzothiazolyl, benzotriazolyl, benzooxazolyl, benzooxadiazolyl, quinazolinyl, quinoxalinyl, carbazolyl, quinolinyl, dibenzofuranyl, dibenzothienyl, furyl (furanyl), imidazolyl, imidazothiazolyl, indazolyl, indolizinyl, indolyl, isoquinolinyl, isoxazoyl, isothiazolyl, indolyl, naphthyridinyl, oxazolyl, oxadiazolyl, phenazinyl, phenothiazinyl, phthalazinyl, pyrazolyl, pyridyl (2-pyridyl, 3-pyridyl, 4-pyridyl), pyrrolyl, pyridazinyl, pyrimidinyl, pyrazinyl, purinyl, phenazinyl, thienyl (thiophenyl), triazolyl, tetrazolyl, thiazolyl, thiadiazolyl and triazinyl.

The term "bridged via a C₁₋₈ aliphatic group" with respect to residues such as aryl, heteroaryl, a heterocycloaliphatic residue, a cycloaliphatic residue or (O-(CH₂)₁₋₃)₀₋₃-O-(CH₂)₀₋₃-CH₃ mean for the purpose of the invention that each of these residues is bound to the respective superordinate general structure via a C₁₋₈ aliphatic group which can be branched or unbranched, unsubstituted or mono- or polysubstituted. Preferred C₁₋₈ alkylene groups are selected from the group consisting of -CH₂-, -CH₂-CH₂-, -CH(CH₃)-, -CH₂-CH₂-CH₂-, -CH(CH₃)-CH₂-, -CH(CH₂CH₃)-, -CH₂-(CH₂)₂-CH₂-, -CH(CH₃)-CH₂-CH₂-, -CH₂-CH(CH₃)-CH₂-, -CH(CH₃)-CH(CH₃)-, -CH(CH₂CH₃)-CH₂-, -C(CH₃)₂-CH₂-, -CH(CH₂CH₂CH₃)-, -C(CH₃)(CH₂CH₃)-, -CH₂-(CH₂)₃-CH₂-, -CH(CH₃)-CH₂-CH₂-CH₂-, -CH₂-CH(CH₃)-CH₂-CH₂-, -CH(CH₃)-CH₂-CH(CH₃)-, -CH(CH₃)-CH(CH₃)-CH₂-, -C(CH₃)₂-CH₂-CH₂-, -CH₂-C(CH₃)₂-CH₂-, -CH(CH₂CH₃)-CH₂-CH₂-, -CH₂-CH(CH₂CH₃)-CH₂-, -C(CH₃)₂-CH(CH₃)-, -CH(CH₂CH₃)-CH(CH₃)-, -C(CH₃)(CH₂CH₃)-CH₂-, -CH(CH₂CH₂CH₃)-CH₂-, -C(CH₂CH₂CH₃)-CH₂-, -CH(CH₂CH₂CH₂CH₃)-, -C(CH₃)(CH₂CH₂CH₃)-, -C(CH₂CH₃)₂- and -CH₂-(CH₂)₄-CH₂-. Particularly preferred C₁₋₈ aliphatic groups are -CH₂-, -CH₂-CH₂- and -CH₂-CH₂-CH₂-.

In relation to the terms "aliphatic residue", "aliphatic group", "cycloaliphatic residue" and "heterocycloaliphatic residue", the term "mono- or polysubstituted" refers in the sense of this invention, with respect to the corresponding residues or groups, to the single substitution or multiple substitution, e.g. disubstitution, trisubstitution, tetrasubstitution, or pentasubstitution, of one or more hydrogen atoms each independently of one another by at least one substituent selected from the group consisting of F; Cl; Br; I; CN; CH₃; C₂₋₄-alkyl; CF₃; CF₂H; CFH₂; CF₂Cl; CFCl₂; C(=O)-H; C(=O)-(C₁₋₄-alkyl); C(=O)-OH;C(=O)-O-(C₁₋₄-alkyl); C(=O)-NH₂; C(=O)-N(H)(C₁₋₄-alkyl); C(=O)-N(C₁₋₄-alkyl)₂; OH; OCH₃; O-C₂₋₄-alkyl; OCF₃; OCF₂H; OCFH₂; OCF₂Cl; OCFCl₂; O-S(=O)₂-OH; O-S(=O)₂-O-C₁₋₄-alkyl; 0-S(=O)₂-NH₂; O-S(=O)₂-N(H)(C₁₋₄-alkyl); O-S(=O)₂-N(C₁₋₄-alkyl)₂; NH₂; NO₂; N(H)(C₁₋₄-alkyl); N(C₁₋₄-alkyl)₂; NH-C(=O)-NH₂; NH-C(=O)-N(H)(C₁₋₄-alkyl); NH-C(=O)-N(C₁₋₄-alkyl)₂; NH-S(=O)₂-OH; NH-S(=O)₂-O-C₁₋₄-alkyl; NH-S(=O)₂-NH₂; NH-S(=O)₂-N(H)(C₁₋₄-alkyl); NH-S(=O)₂-N(C₁₋₄-alkyl)₂; SH; SCH₃; S-C₂₋₄-alkyl; SCF₃; SCF₂H; SCFH₂; SCF₂Cl; SCFCl₂; S(=O)₂-OH; S(=O)₂-O-C₁₋₄-alkyl; S(=O)₂-NH₂; S(=O)₂-N(H)(C₁₋₄-akyl); or S(=O)₂-N(C₁₋₄-alkyl)₂. The term "polysubstituted" with respect to polysubstituted residues and groups includes the polysubstitution of these residues and groups either on different or on the same atoms, for example trisubstituted on the same carbon atom, as in the case of CF₃, CH₂CF₃ or 1,1-difluorocyclohexyl, or at various points, as in the case of CH(OH)-CH=CH-CHCl₂ or 1-chloro-3-fluorocyclohexyl.

In relation to the terms "aryl" and "heteroaryl", the term "mono- or polysubstituted" refers in the sense of this invention, with respect to the corresponding residues or groups, to the single substitution or multiple substitution, e.g. disubstitution, trisubstitution, tetrasubstitution, or pentasubstitution, of one or more hydrogen atoms each independently of one another by at least one substituent selected from the group consisting of F; Cl; Br; I; CN; CH₃; C₂₋₄-alkyl; CF₃; CF₂H; CFH₂; CF₂Cl; CFCl₂; C(=O)-H; C(=O)-(C₁₋₄-alkyl); C(=O)-OH;C(=O)-O-(C₁₋₄-alkyl); C(=O)-NH₂; C(=O)-N(H)(C₁₋₄-alkyl); C(=O)-N(C₁₋₄-alkyl)₂; OH; OCH₃; O-C₂₋₄-alkyl; OCF₃; OCF₂H; OCFH₂; OCF₂Cl; OCFCl₂; O-S(=O)₂-OH; O-S(=O)₂-O-C₁₋₄-alkyl; O-S(=O)₂-NH₂; O-S(=O)₂-N(H)(C₁₋₄-alkyl); O-S(=O)₂-N(C₁₋₄-alkyl)₂; NH₂; NO₂; N(H)(C₁₋₄-alkyl); N(C₁₋₄-alkyl)₂; NH-C(=O)-NH₂; NH-C(=O)-N(H)(C₁₋₄-alkyl); NH-C(=O)-N(C₁₋₄-alkyl)₂; NH-S(=O)₂-OH; NH-S(=O)₂-O-C₁₋₄-alkyl; NH-S(=O)₂-NH₂; NH-S(=O)₂-N(H)(C₁₋₄-alkyl); NH-S(=O)₂-N(C₁₋₄-alkyl)₂; SH; SCH₃; S-C₂₋₄-alkyl; SCF₃; SCF₂H; SCFH₂; SCF₂Cl; SCFCl₂; S(=O)₂-OH; S(=O)₂-O-C₁₋₄-alkyl; S(=O)₂-NH₂; S(=O)₂-N(H)(C₁₋₄-alkyl); or S(=O)₂-N(C₁₋₄-alkyl)₂.

The compounds according to the present invention are defined by substituents, for example by R¹, R² and R³ (1^{st} generation substituents) which if appropriate are substituted themselves (2^{nd} generation substituents). However, preferably, the 2^{nd} generation substituents may not be substituted.

Within the scope of the present invention, the symbol 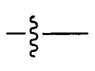 used in the formulae denotes a link of a corresponding residue to the respective superordinate general structure.

If a residue occurs multiply within a molecule, then this residue can have respectively different meanings for various substituents: if, for example, both R¹ and R² denote a 3 to 6 membered heterocycloaliphatic residue, then the 3 to 6 membered heterocycloaliphatic residue can e.g. represent morpholinyl for R¹ and can represent piperazinyl for R².

If a residue occurs multiply within a molecule or substituent, such as e.g. the residue C₁₋₄-alkyl, then this residue can have respectively different meanings within the scope of its definition.

The terms "salt formed with a physiologically compatible acid" or "salt of physiologically acceptable acids" refers in the sense of this invention to salts of the respective active ingredient with inorganic or organic acids which are physiologically compatible - in particular when used in human beings and/or other mammals.

The terms "salt formed with a physiologically compatible base" or "salt of physiologically acceptable bases" refers in the sense of this invention to salts of the respective compound according to the invention - as an anion, e.g. upon deprotonation of a suitable functional group - with at least one cation or base - preferably with at least one inorganic cation - which are physiologically acceptable - in particular when used in human beings and/or other mammals.
According to the present invention, n represents 1, 2, 3 or 4; preferably 1, 2 or 3 and more preferably 1 or 2. In a particularly preferred embodiment, n represents 1.

According to the present invention, Y represents O, S or N-CN. Most preferably, Y represents O.

In a preferred embodiment, R³ is selected from the group consisting of H, CH₃ and CH₂CH₃. More preferably, R³ represents H.

According to the present invention, Z represents N or CR^{4b}. In a preferred embodiment, Z represents CR^{4b}. According to this embodiment, R^{4b} preferably stands for H, CH₃ or CH₂CH₃, more preferably, R^{4b} stands for H. In another preferred embodiment, Z represents N.

In a preferred embodiment, R^{4a} represents H, CH₃, CH₂CH₃, cyclopropyl, cyclobutyl, cyclopentyl, cyclohexyl, or phenyl, wherein phenyl is unsubstituted or substituted with 1, 2, 3, 4 or 5 substituents independently selected from the group consisting of F, Cl, Br, I, NO₂, CN, CF₃, CF₂H, CFH₂, CF₂Cl, CFCl₂, OH, NH₂, NH(C₁₋₄ alkyl) and N(C₁₋₄ alkyl)(C₁₋₄ alkyl), C₁₋₄ alkyl, and O-C₁₋₄-alkyl; and R^{4b} represents H, CH₃ or CH₂CH₃.

In another preferred embodiment, R^{4a} represents H, CH₃ or CH₂CH₃; more preferably H or CH₃.

According to the present invention, X represents N or CH. Preferably, X represents N.

In a particularly preferred embodiment, n represents 1, Y represents O, R³ represents H and X represents N.

In a preferred embodiment, R¹ is selected from the group consisting of CF₃, CH₃, CH₂CH₃, (CH₂)₂CH₃, CH(CH₃)₂, (CH₂)₃CH₃, CH(CH₃)CH₂CH₃, and C(CH₃)₃ or is selected from the group consisting of cyclopropyl, cyclobutyl, cyclopentyl, and cyclohexyl.

In another preferred embodiment, R¹ is selected from the group consisting of CF₃, CH₃ or cyclopropyl, more preferably R¹ represents CF₃.

In a preferred embodiment, R² represents substructure (T) in which 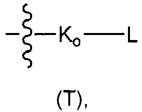
- K: represents O, an unsubstituted C₁₋₆ aliphatic group or NR⁸, wherein R⁸ represents H or a C₁₋₄ aliphatic residue, unsubstituted or mono- or polysubstituted with one or more substituents each selected independently of one another from the group consisting of F, Cl, Br, I and C₁₋₄ alkyl;
- o: represents 0 or 1;
- L: represents an unsubstituted or mono- or polysubstituted C₃₋₆-cycloaliphatic residue,
an unsubstituted or mono- or polysubstituted 3 to 6-membered heterocycloaliphatic residue containing 1 to 3 heteroatoms, wherein binding to K occurs via a heteroatom or a carbon atom,
an unsubstituted or mono- or polysubstituted aryl,
an unsubstituted or mono- or polysubstituted heteroaryl containing 1 to 3 heteroatoms, wherein binding to K occurs via a heteroatom or a carbon atom,
(O-(CH₂)₁₋₃)₀₋₃-O-(CH₂)₀₋₃-CH₃,
N(H)-C₁₋₆ aliphatic residue, unsubstituted or mono- or polysubstituted,
in which "mono- or polysubstituted" with respect to the aforementioned aliphatic residue, cycloaliphatic residue, heterocycloaliphatic residue, aryl and heteroaryl relates in each case independently of one another, with respect to the corresponding residues, to the substitution of one or more hydrogen atoms each independently of one another by at least one substituent selected from the group consisting of F; Cl; Br; I; CN; CH₃; C₂₋₄-alkyl; CF₃; OH; OCH₃; O-C₂₋₄-alkyl; OCF₃; OCF₂H; OCFH₂; NO₂; N(H)(C₁₋₄-alkyl); N(C₁₋₄-alkyl)₂.

In a preferred embodiment, when L represents a 3 to 6-membered heterocycloaliphatic residue or a heteroaryl, binding to K occurs via the heteroatom. In another preferred embodiment, when L represents a 3 to 6-membered heterocycloaliphatic residue or a heteroaryl, binding to K occurs via the carbon atom.

In a preferred embodiment, K represents O or an unsubstituted C₁₋₃ aliphatic group, more preferably O, methylene, ethylene or propylene.

In another preferred embodiment, o represents 0, i.e. group K is not present and R² is defined by L.

Preferably, L is selected from
cyclopropyl, cylcobutyl, cyclopentyl and cyclohexyl;
pyrrolidinyl (tetrahydropyrrolyl), piperidinyl, tetrahydropyranyl, morpholinyl, tetrahydrofuranyl, tetrahydrothiophenyl, tetrahydrothiopyran, imidazolidinyl, piperazinyl, 4-methylpiperazinyl, tetrahydro-2H-pyran-4-yl, tetrahydroquinolinyl, tetrahydroisoquinolinyl, dihydroquinolinyl, dihydropyrrolyl, dihydropyridinyl, dihydroisoquinolinyl, tetrahydropyridinyl and thiomorpholinyl, azetidinyl, aziridinyl, dithiolanyl, dioxanyl, dioxolanyl, dihydrofuranyl, isoxazolidinyl, oxiranyl, pyrazolidinyl, pyranyl, tetrahydropyridinyl, tetrahydroisoxazololyl, thiazolidinyl;
phenyl;
pyridyl (1-pyridyl, 2-pyridyl, 3-pyridyl, 4-pyridyl), pyrrolyl, thienyl (thiophenyl), furanyl, pyrazolyl, imidazolyl, indolyl, quinolinyl, isoquinolinyl, purinyl, pyrimidinyl, oxazolyl, thiazolyl, benzofuranyl, benzoimidazolyl, benzothienyl, benzothiadiazolyl, benzothiazolyl, benzotriazolyl, benzooxazolyl, benzooxadiazolyl, quinazolinyl, quinoxalinyl, carbazolyl, quinolinyl, dibenzofuranyl, dibenzothienyl, imidazothiazolyl, indazolyl, indolizinyl, isoxazoyl, isothiazolyl, naphthyridinyl, oxadiazolyl, phenazinyl, phenothiazinyl, phthalazinyl, pyridazinyl, pyrazinyl, purinyl, triazolyl, tetrazolyl, thiadiazolyl and triazinyl;
N(H)-C₁₋₆ aliphatic residue; and
(O-(CH₂)₁₋₃)₀₋₃-O-(CH₂)₀₋₃-CH₃;
in each case, where appropriate, unsubstituted or mono- or polysubstituted with one or more substituents each selected independently of one another from the group consisting of F, Cl, Br, I, CH₃, C₂₋₄-alkyl, CF₃, OH, OCH₃, O-C₂₋₄-alkyl, OCF₃, N(H)(C₁₋₄ alkyl), N(C₁₋₄ alkyl)₂.

In another preferred embodiment, R² represents substructure (T) in which 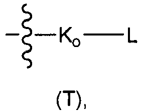
- K: represents O or an unsubstituted C₁₋₃ aliphatic group;
- o: represents 0 or 1;
- L: represents an unsubstituted or monosubstituted C₅₋₆-cycloaliphatic residue,
an unsubstituted or monosubstituted 5 to 6-membered heterocycloaliphatic residue containing 1 or 2 heteroatoms, wherein binding to K occurs via a heteroatom or a carbon atom,
an unsubstituted or monosubstituted 5 to 6-membered aryl,
an unsubstituted or monosubstituted 5 to 6-membered heteroaryl containing 1 to 3 heteroatoms, wherein binding to K occurs via a heteroatom or a carbon atom,
(O-(CH₂)₁₋₃)₀₋₃-O-(CH₂)₀₋₃-CH₃,
N(H)-C₁₋₆ unsubstituted aliphatic residue,
in which "monosubstituted" with respect to the aforementioned residues relates in each case independently of one another, with respect to the corresponding residues, to the substitution of one hydrogen atom by one substituent selected from the group consisting of CH₃, CH₂CH₃, (CH₂)₂CH₃, CH(CH₃)₂, (CH₂)₃CH₃, CH(CH₃)CH₂CH₃, and C(CH₃)₃ and CF₃.

Most preferably, L is selected from cyclopentyl, cyclohexyl, pyrrolidin-1-yl, piperidin-1-yl, 4-methylpiperidin-1-yl, tetrahydro-2H-pyran-4-yl, N-morpholinyl, 3-methyl-phenyl, unsubstituted NH-C₁₋₆ aliphatic residue and (O-(CH₂)₁₋₃)₀₋₃-O-(CH₂)₀₋₃-CH₃.

In a particularly preferred embodiment, R² represents cyclopentyl, (cyclohexyl)methyl, pyrrolidin-1-yl, piperidin-1-yl, 4-methylpiperidin-1-yl, tetrahydro-2H-pyran-4-yl, O-tetrahydro-2H-pyran-4-yl, N-morpholinyl, 3-methyl-phenyl, N(H)(CH₂CH₃), N(H)((CH₂)₃CH₃), (CH₂)₃-O-CH₃, CH₂-O-(CH₂)₂-O-CH₃.

In a preferred embodiment, 1 of variables A² and A³, respectively, represents N. According to this embodiment, preferably A³ represents N, A² represents CR⁷.

In a preferred embodiment, R⁶ represents H; CH₃; CH₂CH₃; (CH₂)₂CH₃; CH(CH₃)₂; (CH₂)₃CH₃; cyclopentyl or cyclohexyl, in each case unsubstituted. According to this embodiment, R⁶ preferably represents H, CH₃ or CH₂CH₃ and most preferably H or CH₃.

In a preferred embodiment, represents bond, H, F, Cl, Br, I, CH₃; CH₂CH₃; (CH₂)₂CH₃; CH(CH₃)₂; (CH₂)₃CH₃; in each case, where appropriate, unsubstituted. According to this embodiment, R⁷ preferably represents bond, H or CH₃ and most preferably bond or H.

According to the present invention, partial structure (E) 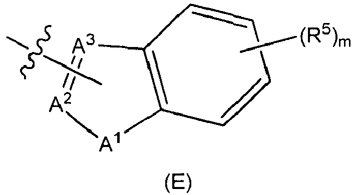 represents an aromatic heterocyclic residue comprising an aromatic 5-membered heterocyclic residue which is condensed with a phenyl residue. Thus, Z is bound to one of the atoms forming the aromatic 5-membered heterocyclic residue contained in partial structure (E).

In a preferred embodiment, the compound according to the present invention is characterized in that the partial structure (E) represents a moiety selected from the group consisting of: 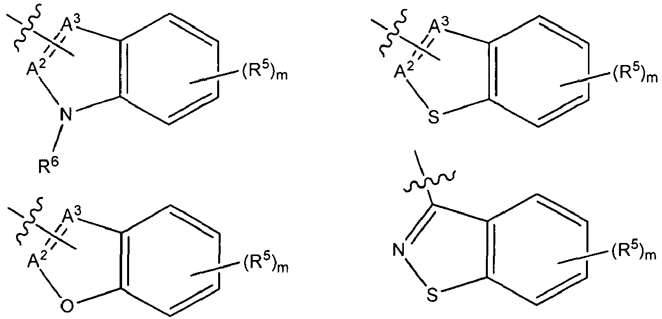 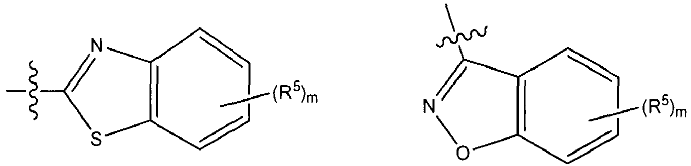 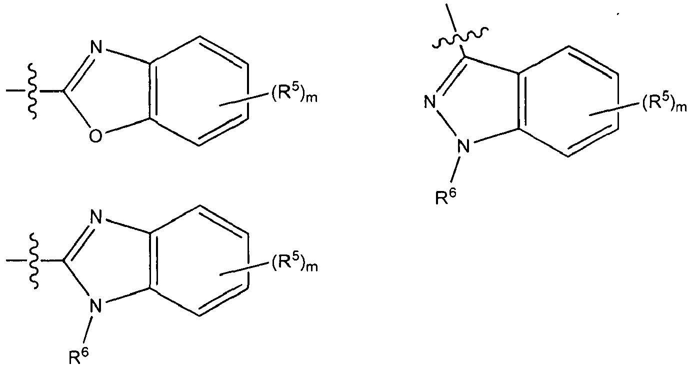 wherein
- A² and A³: represent CR⁷ with representing bond, H, CH₃ or C₂₋₄-alkyl.

Preferably, m represents 0, 1, 2 or 3; more preferably 0, 1 or 2 and most preferably 0 or 1. In a preferred embodiment, m is 0. In another preferred embodiment, m is 1.

In a preferred embodiment, R⁵ each independently of one another represents OH, OCH₃, NO₂, F, Cl, Br or I; most preferably OH, OCH₃, Cl or NO₂. According to this embodiment, m is preferably 1.

In another preferred embodiment,
- m: is 0 or 1 and
- R⁵: represents F; Cl; Br; I; CN; CH₃; CF₃; OH; OCH₃; O-C₂₋₄-alkyl; OCF₃; NO₂.

In a particularly preferred embodiment, substructure (E) is selected from the group consisting of indolyl, benzothiazolyl and benzimidazolyl. According to this embodiment, m preferably represents 0 or 1.

Preferably, the compound according to the present invention is characterized in that
- n: represents 1;
- Y: represents 0;
- R¹: represents CF₃, CH₃ or cyclopropyl;
- X: represents N;
- R³: represents H;
- Z: represents N or CR^{4b}, wherein R^{4b} represents H or CH₃;
- R^{4a}: represents H or CH₃;
- R²: represents substructure (T) in which 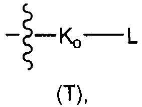
- K: represents O or an unsubstituted C₁₋₃aliphatic group;
- o: represents 0 or 1;
- L: represents an unsubstituted or monosubstituted C₅₋₆-cycloaliphatic residue,
an unsubstituted or monosubstituted 5 to 6-membered heterocycloaliphatic residue containing 1 or 2 heteroatoms, wherein binding to K occurs via a heteroatom or a carbon atom,
an unsubstituted or monosubstituted 5 to 6-membered aryl,
an unsubstituted or monosubstituted 5 to 6-membered heteroaryl containing 1 to 3 heteroatoms, wherein binding to K occurs via a heteroatom or a carbon atom,
(O-(CH₂)₁₋₃)₀₋₃-O-(CH₂)₀₋₃-CH₃,
NH-C₁₋₆ unsubstituted aliphatic residue,
in which "monosubstituted" with respect to the aforementioned residues relates in each case independently of one another, with respect to the corresponding residues, to the substitution of one hydrogen atom by one substituent selected from the group consisting of CH₃, CH₂CH₃, (CH₂)₂CH₃, CH(CH₃)₂, (CH₂)₃CH₃, CH(CH₃)CH₂CH₃, C(CH₃)₃ and CF₃;
partial structure (E) is selected from the group consisting of 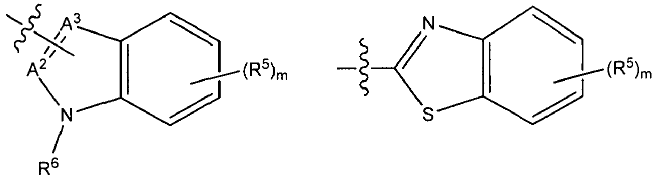 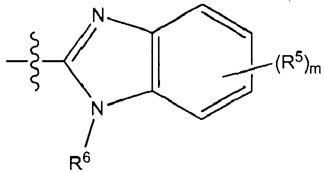 wherein
- A² and A³: represent CR⁷ with R⁷ representing bond or H;
- m: represents 0 or 1, and
- R⁵: represents OH; OCH₃; NO₂; F; Cl; Br or I.

Further embodiments of the compounds according to the present invention are those which are represented by the general formulae A1, A2 and A3 shown in the following: 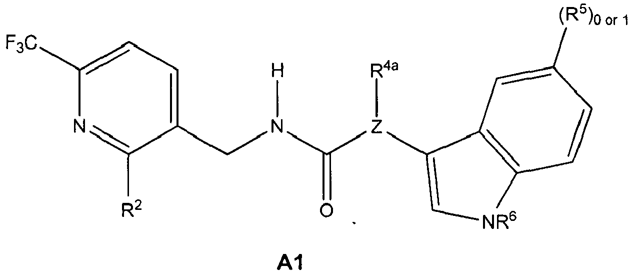 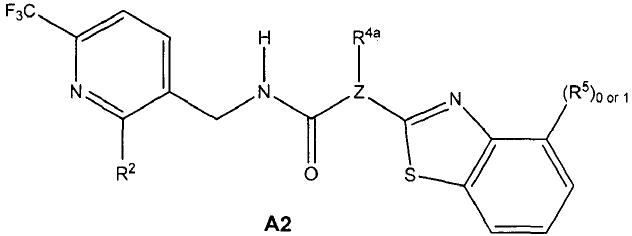 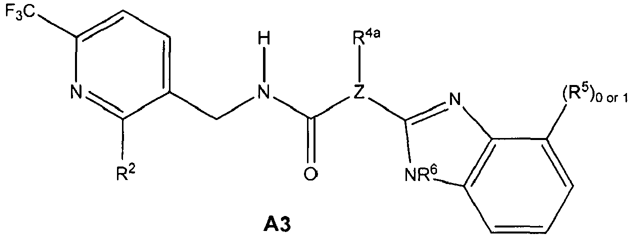 wherein the particular radicals, variables and indices have the meanings described herein in connection with the compounds according to the invention and preferred embodiments thereof;
with the proviso that when R⁵ is not present, i.e. "(R⁵)₀", the respective position in the ring is substituted with H.

Particularly preferred compounds according to the present invention are those which are represented by the general formula A1 shown above, wherein Z représents CH, R^{4a} represents H or CH₃, R⁶ represents CH₃ and R⁵ represents OH or OCH₃.

In a preferred embodiment, the compound according to the present invention is selected from the group consisting of
1 2-(5-hydroxy-1-methyl-1H-indol-3-yl)-N-((2-(4-methylpiperidin-1-yl)-6-(trifluoromethyl)pyridin-3-yl)methyl)propanamide
2 2-(5-methoxy-1-methyl-1H-indol-3-yl)-N-((2-(4-methylpiperidin-1-yl)-6-(trifluoromethyl)pyridin-3-yl)methyl)acetamide
3 N-((2-cyclopentyl-6-(trifluoromethyl)pyridin-3-yl)methyl)-2-(5-hydroxy-1-methyl-1H-indol-3-yl)propanamide
4 N-((2-(ethylamino)-6-(trifluoromethyl)pyridin-3-yl)methyl)-2-(5-hydroxy-1-methyl-1H-indol-3-yl)propanamide
5 2-(5-hydroxy-1-methyl-1H-indol-3-yl)-N-((2-(pyrrolidin-1-yl)-6-(trifluoromethyl)pyridin-3-yl)methyl)propanamide
6 N-((2-(butylamino)-6-(trifluoromethyl)pyridin-3-yl)methyl)-2-(5-hydroxy-1-methyl-1 H-indol-3-yl)propanamide
7 2-(5-hydroxy-1-methyl-1H-indol-3-yl)-N-((2-((tetrahydro-2H-pyran-4-yl)oxy)-6-(trifluoromethyl)pyridin-3-yl)methyl)propanamide
8 2-(5-hydroxy-1-methyl-1H-indol-3-yl)-N-((2-(m-tolyl)-6-(trifluoromethyl)pyridin-3-yl)methyl)propanamide
9 2-(5-hydroxy-1-methyl-1H-indol-3-yl)-N-((2-(tetrahydro-2H-pyran-4-yl)-6-(trifluoromethyl)pyridin-3-yl)methyl)propanamide
10 2-(5-hydroxy-1-methyl-1 H-indol-3-yl)-N-((2-morpholino-6-(trifluoromethyl)pyridin-3-yl)methyl)propanamide
11 2-(5-hydroxy-1-methyl-1H-indol-3-yl)-N-((2-(4-methylpiperidin-1-yl)-6-(trifluoromethyl)pyridin-3-yl)methyl)acetamide
12 2-(5-hydroxy-1-methyl-1H-indol-3-yl)-N-((2-(3-methoxypropyl)-6-(trifluoromethyl)pyridin-3-yl)methyl)propanamide
13 2-(5-hydroxy-1-methyl-1H-indol-3-yl)-N-((2-(piperidin-1-yl)-6-(trifluoromethyl)pyridin-3-yl)methyl)propanamide
14 2-(1-methyl-1H-indol-3-yl)-N-((2-(4-methylpiperidin-1-yl)-6-(trifluoromethyl)pyridin-3-yl)methyl)propanamide
15 2-(5-hydroxy-1-methyl-1H-indol-3-yl)-N-((2-((2-methoxyethoxy)methyl)-6-(trifluoromethyl)pyridin-3-yl)methyl)propanamide
**16** N-((2-(ethylamino)-6-(trifluoromethyl)pyridin-3-yl)methyl)-2-(5-methoxy-1-methyl-1H-indol-3-yl)propanamide
**17** N-((2-(ethylamino)-6-(trifluoromethyl)pyridin-3-yl)methyl)-2-(5-methoxy-1-methyl-1H-indol-3-yl)acetamide
**18** N-((2-(ethylamino)-6-(trifluoromethyl)pyridin-3-yl)methyl)-2-(5-hydroxy-1-methyl-1 H-indol-3-yl)acetamide
**19** N-((2-(butylamino)-6-(trifluoromethyl)pyridin-3-yl)methyl)-2-(5-methoxy-1-methyl-1H-indol-3-yl)propanamide
**20** 2-(5-hydroxy-1-methyl-1H-indol-3-yl)-N-((2-morpholino-6-(trifluoromethyl)pyridin-3-yl)methyl)acetamide
**21** N-((2-cyclopentyl-6-(trifluoromethyl)pyridin-3-yl)methyl)-2-(5-hydroxy-1-methyl-1H-indol-3-yl)acetamide
**22** N-((2-(cyclohexylmethyl)-6-(trifluoromethyl)pyridin-3-yl)methyl)-2-(1-methyl-1H-indol-3-yl)propanamide
**23** 1-(4-chlorobenzo[d]thiazol-2-yl)-3-((2-(4-methylpiperidin-1-yl)-6-(trifluoromethyl)pyridin-3-yl)methyl)urea
**24** 1-((2-(4-methylpiperidin-1-yl)-6-(trifluoromethyl)pyridin-3-yl)methyl)-3-(4-nitrobenzo[d]thiazol-2-yl)urea
**25** 1-(benzo[d]thiazol-2-yl)-3-((2-(4-methylpiperidin-1-yl)-6-(trifluoromethyl)pyridin-3-yl)methyl)urea
**26** 1-(1H-benzo[d]imidazol-2-yl)-3-((2-(4-methylpiperidin-1-yl)-6-(trifluoromethyl)pyridin-3-yl)methyl)urea
optionally in the form of a single stereoisomer or a mixture of stereoisomers, in the form of the free compound and/or a physiologically acceptable salt thereof.

According to this embodiment, more preferably the compound according to the present invention is selected from the group consisting of compounds **1** to **15, 17** to **22, 24** and **25.**

Furthermore, preference may be given to compounds according to the invention that cause a 50 per cent displacement of capsaicin, which is present at a concentration of 100 nM, in a FLIPR assay with CHO K1 cells which were transfected with the human VR1 gene at a concentration of less than 6,000 nM, preferably less than 5,000 nM, more preferably less that 3,000 nM or less than 1,000 nM and particularly preferably less than 300 nM, most particularly preferably less than 100 nM, even more preferably less than 75 nM, additionally preferably less than 50 nM, most preferably less than 10 nM.

In the process, the Ca²⁺ influx is quantified in the FLIPR assay with the aid of a Ca²⁺-sensitive dye (type Fluo-4, Molecular Probes Europe BV, Leiden, the Netherlands) in a fluorescent imaging plate reader (FLIPR, Molecular Devices, Sunnyvale, USA), as described hereinafter.

The compounds according to the present invention and the corresponding stereoisomers and also the respective corresponding acids, bases, salts and solvates are toxicologically safe and are therefore suitable as pharmaceutical active ingredients in pharmaceutical compositions.

The present invention therefore further relates to a pharmaceutical composition comprising at least one compound according to the present invention.

The pharmaceutical composition can contain the compound according to the present invention, if appropriate, in the form of one of its pure stereoisomers, in particular enantiomers or diastereomers, its racemates or in the form of a mixture of stereoisomers, in particular the enantiomers and/or diastereomers, in any desired mixing ratio, or respectively in the form of a corresponding salt, or respectively in the form of a corresponding solvate, and also if appropriate one or more pharmaceutically compatible auxiliaries.

These pharmaceutical compositions according to the invention are suitable in particular for vanilloid receptor 1-(VR1/TRPV1) regulation, preferably for vanilloid receptor 1-(VR1/TRPV1) inhibition and/or for vanilloid receptor 1-(VR1/TRPV1) stimulation, i.e. they exert an agonistic or antagonistic effect.

Likewise, the pharmaceutical compositions according to the invention are preferably suitable for the prophylaxis and/or treatment of disorders or diseases which are mediated, at least in part, by vanilloid receptors 1.

The pharmaceutical composition according to the invention is suitable for administration to adults and children, including toddlers and babies.

The pharmaceutical composition according to the invention may be found as a liquid, semisolid or solid pharmaceutical form, for example in the form of injection solutions, drops, juices, syrups, sprays, suspensions, tablets, patches, capsules, plasters, suppositories, ointments, creams, lotions, gels, emulsions, aerosols or in multiparticulate form, for example in the form of pellets or granules, if appropriate pressed into tablets, decanted in capsules or suspended in a liquid, and also be administered as much.

In addition to at least one compound of formula (I), if appropriate in the form of one of its pure stereoisomers, in particular enantiomers or diastereomers, its racemate or in the form of mixtures of the stereoisomers, in particular the enantiomers or diastereomers, in any desired mixing ratio, or if appropriate in the form of a corresponding salt or respectively in the form of a corresponding solvate, the pharmaceutical composition according to the invention conventionally contains further physiologically compatible pharmaceutical auxiliaries which can for example be selected from the group consisting of excipients, fillers, solvents, diluents, surface-active substances, dyes, preservatives, blasting agents, slip additives, lubricants, aromas and binders.

The selection of the physiologically compatible auxiliaries and also the amounts thereof to be used depend on whether the pharmaceutical composition is to be applied orally, subcutaneously, parenterally, intravenously, intraperitoneally, intradermally, intramuscularly, intranasally, buccally, rectally or locally, for example to infections of the skin, the mucous membranes and of the eyes. Preparations in the form of tablets, dragées, capsules, granules, pellets, drops, juices and syrups are preferably suitable for oral application; solutions, suspensions, easily reconstitutable dry preparations and also sprays are preferably suitable for parenteral, topical and inhalative application. The compounds according to the present invention used in the pharmaceutical composition according to the present invention in a repository in dissolved form or in a plaster, agents promoting skin penetration being added if appropriate, are suitable percutaneous application preparations. Orally or percutaneously applicable preparation forms can release the respective compound according to the present invention also in a delayed manner.

The pharmaceutical compositions according to the present invention are prepared with the aid of conventional means, devices, methods and process known in the art, such as are described for example in "Remington's Pharmaceutical Sciences", A.R. Gennaro (Editor), 17^{th} edition, Mack Publishing Company, Easton, Pa, 1985, in particular in Part 8, Chapters 76 to 93. The corresponding description is introduced herewith by way of reference and forms part of the disclosure. The amount to be administered to the patient of the respective compounds according to the invention of the above-indicated general formula I may vary and is for example dependent on the patient's weight or age and also on the type of application, the indication and the severity of the disorder. Conventionally 0.001 to 100 mg/kg, preferably 0.05 to 75 mg/kg, particularly preferably 0.05 to 50 mg of at least one such compound according to the invention are applied per kg of the patient's body weight.

Another aspect of the present inventions relates to a compound according to the present invention for use in the treatment and/or prophylaxis of one or more diseases and/or disorders selected from the group consisting of pain, preferably of pain selected from the group consisting of acute pain, chronic pain, neuropathic pain, visceral pain and joint pain; hyperalgesia; allodynia; causalgia; migraine; depression; nervous affection; axonal injuries; neurodegenerative diseases, preferably selected from the group consisting of multiple sclerosis, Alzheimer's disease, Parkinson's disease and Huntington's disease; cognitive dysfunctions, preferably cognitive deficiency states, particularly preferably memory disorders; epilepsy; respiratory diseases, preferably selected from the group consisting of asthma, bronchitis and pulmonary inflammation; coughs; urinary incontinence; overactive bladder (OAB); disorders and/or injuries of the gastrointestinal tract; duodenal ulcers; gastric ulcers; irritable bowel syndrome; strokes; eye irritations; skin irritations; neurotic skin diseases; allergic skin diseases; psoriasis; vitiligo; herpes simplex; inflammations, preferably inflammations of the intestine, the eyes, the bladder, the skin or the nasal mucous membrane; diarrhoea; pruritus; osteoporosis; arthritis; osteoarthritis; rheumatic diseases; eating disorders, preferably selected from the group consisting of bulimia, cachexia, anorexia and obesity; medication dependency; misuse of medication; withdrawal symptoms in medication dependency; development of tolerance to medication, preferably to natural or synthetic opioids; drug dependency; misuse of drugs; withdrawal symptoms in drug dependency; alcohol dependency; misuse of alcohol and withdrawal symptoms in alcohol dependency; for diuresis; for antinatriuresis; for influencing the cardiovascular system; for increasing vigilance; for the treatment of wounds and/or burns; for the treatment of severed nerves; for increasing libido; for modulating movement activity; for anxiolysis; for local anaesthesia and/or for inhibiting undesirable side effects, preferably selected from the group consisting of hyperthermia, hypertension and bronchoconstriction, triggered by the administration of vanilloid receptor 1 (VR1/TRPV1 receptor) agonists, preferably selected from the group consisting of capsaicin, resiniferatoxin, olvanil, arvanil, SDZ-249665, SDZ-249482, nuvanil and capsavanil.

According to this embodiment, the compound according to the present invention can optionally be combined with one or more pharmaceutically acceptable auxiliaries.

Particularly preferably, the pharmaceutical composition according to the invention is suitable for use in the treatment and/or prophylaxis of one or more disorders and/or diseases selected from the group consisting of pain, preferably of pain selected from the group consisting of acute pain, chronic pain, neuropathic pain, visceral pain and joint pain; migraine; depression; neurodegenerative diseases, preferably selected from the group consisting of multiple sclerosis, Alzheimer's disease, Parkinson's disease and Huntington's disease; cognitive dysfunctions, preferably cognitive deficiency states, particularly preferably memory disorders; inflammations, preferably inflammations of the intestine, the eyes, the bladder, the skin or the nasal mucous membrane; urinary incontinence; overactive bladder (OAB); medication dependency; misuse of medication; withdrawal symptoms in medication dependency; development of tolerance to medication, preferably development of tolerance to natural or synthetic opioids; drug dependency; misuse of drugs; withdrawal symptoms in drug dependency; alcohol dependency; misuse of alcohol and withdrawal symptoms in alcohol dependency.

Most particularly preferably, the pharmaceutical composition according to the invention is suitable for use in the treatment and/or prophylaxis of pain, preferably of pain selected from the group consisting of acute pain, chronic pain, neuropathic pain and visceral pain.

The present invention further relates to a compound according to formula (I) and also, if appropriate, to a compound according to formula (I) and one or more pharmaceutically acceptable auxiliaries for use in vanilloid receptor 1-(VR1/TRPV1) regulation, preferably for use in vanilloid receptor 1-(VR1/TRPV1) inhibition and/or vanilloid receptor 1-(VR1/TRPV1) stimulation.

The present invention therefore further relates to a compound according to formula (I) and also if appropriate to a compound according to formula (I) and one or more pharmaceutically acceptable auxiliaries for use in the prophylaxis and/or treatment of disorders and/or diseases which are mediated, at least in part, by vanilloid receptors 1.

The present invention further relates to the use of at least one compound according to formula (I) and also, if appropriate, of one or more pharmaceutically acceptable auxiliaries for the preparation of a pharmaceutical composition for vanilloid receptor 1-(VR1/TRPV1) regulation, preferably for vanilloid receptor 1-(VR1/TRPV1) inhibition and/or for vanilloid receptor 1-(VR1/TRPV1) stimulation, and, further for the prophylaxis and/or treatment of disorders and/or diseases which are mediated, at least in part, by vanilloid receptors 1.

Another aspect of the present invention is a method for vanilloid receptor 1-(VR1/TRPV1) regulation, preferably for vanilloid receptor 1-(VR1/TRPV1) inhibition and/or for vanilloid receptor 1-(VR1/TRPV1) stimulation.

Still another aspect of the present invention relates to a method of treatment and/or prophylaxis of disorders and/or diseases selected from the group consisting of pain, preferably of pain selected from the group consisting of acute pain, chronic pain, neuropathic pain, visceral pain and joint pain; hyperalgesia; allodynia; causalgia; migraine; depression; nervous affection; axonal injuries; neurodegenerative diseases, preferably selected from the group consisting of multiple sclerosis, Alzheimer's disease, Parkinson's disease and Huntington's disease; cognitive dysfunctions, preferably cognitive deficiency states, particularly preferably memory disorders; epilepsy; respiratory diseases, preferably selected from the group consisting of asthma, bronchitis and pulmonary inflammation; coughs; urinary incontinence; overactive bladder (OAB); disorders and/or injuries of the gastrointestinal tract; duodenal ulcers; gastric ulcers; irritable bowel syndrome; strokes; eye irritations; skin irritations; neurotic skin diseases; allergic skin diseases; psoriasis; vitiligo; herpes simplex; inflammations, preferably inflammations of the intestine, the eyes, the bladder, the skin or the nasal mucous membrane; diarrhoea; pruritus; osteoporosis; arthritis; osteoarthritis; rheumatic diseases; eating disorders, preferably selected from the group consisting of bulimia, cachexia, anorexia and obesity; medication dependency; misuse of medication; withdrawal symptoms in medication dependency; development of tolerance to medication, preferably to natural or synthetic opioids; drug dependency; misuse of drugs; withdrawal symptoms in drug dependency; alcohol dependency; misuse of alcohol and withdrawal symptoms in alcohol dependency; for diuresis; for antinatriuresis; for influencing the cardiovascular system; for increasing vigilance; for the treatment of wounds and/or burns; for the treatment of severed nerves; for increasing libido; for modulating movement activity; for anxiolysis; for local anaesthesia and/or for inhibiting undesirable side effects, preferably selected from the group consisting of hyperthermia, hypertension and bronchoconstriction, triggered by the administration of vanilloid receptor 1 (VR1/TRPV1 receptor) agonists, preferably selected from the group consisting of capsaicin, resiniferatoxin, olvanil, arvanil, SDZ-249665, SDZ-249482, nuvanil and capsavanil, which comprises administering an effective amount of at least one compound according to the present invention to the mammal.

The effectiveness against pain can be shown, for example, in the *Bennett* or *Chung* model (Bennett, G.J. and Xie, Y.K., A peripheral mononeuropathy in rat that produces disorders of pain sensation like those seen in man, Pain 1988, 33(1), 87-107; Kim, S.H. and Chung, J.M., An experimental model for peripheral neuropathy produced by segmental spinal nerve ligation in the rat, Pain 1992, 50(3), 355-363), by tail flick experiments (e.g. according to D'Amour und Smith (J. Pharm. Exp. Ther. 72, 74 79 (1941)) or by the formalin test (e.g. according to D. Dubuisson et al., Pain 1977, 4, 161-174).

### Examples

### Abbreviations:

AcOH: acetic acid; brine: saturated sodium chloride solution (NaCl sol.); Boc: tert-butyloxycarbonyl; comp. no.: example compound number; EE: ethyl acetate; EtOH: ethanol; h: hour(s); M: mol/L; MeOH: methanol; min: minutes; SC: silica gel column chromatography; RT: room temperature; THF: tetrahydrofuran.

The stationary phase used for the column chromatography was silica gel (100-200 mesh) from E. Merck, Darmstadt.

The yields of the compounds prepared were not optimized and all temperatures are uncorrected.

All starting materials which are not explicitly described were either commercially available (the details of suppliers such as for example Acros, Avocado, Aldrich, Bachem, Fluka, Lancaster, Maybridge, Merck, Sigma, TCI, Oakwood, etc. can be found in the Symyx® Available Chemicals Database of MDL, San Ramon, US, for example) or the synthesis thereof has already been described precisely in the specialist literature (experimental guidelines can be looked up in the Reaxys® Database of Elsevier, Amsterdam, NL, for example) or can be prepared using the conventional methods known to the person skilled in the art.

### Synthesis of the exemplary compounds:

The acids and amines disclosed in Table 1 herein below are commercially available.

**Table 1:**

| | |
|---|---|
| 2-(5-hydroxy-1-methyl-1H-indol-3-yl)propanoic acid | **ACI-1** |
| 2-(5-methoxy-1-methyl-1H-indol-3-yl)acetic acid | **ACI-2** |
| 2-(5-hydroxy-1-methyl-1H-indol-3-yl)acetic acid | **ACI-3** |
| 2-(1-methyl-1H-indol-3-yl)propanoic acid | **ACI-4** |
| 4-chlorobenzo[d]thiazol-2-amine | **AMI-1** |
| 4-nitrobenzo[d]thiazol-2-amine | **AMI-2** |
| benzo[d]thiazol-2-amine | **AMI-3** |
| 1H-benzo[d]imidazol-2-amine | **AMI-4** |

The synthesis of 2-(5-methoxy-1-methyl-1 H-indol-3-yl)propanoic acid **(ACI-5)** has been described in J. Med. Chem. 2006, 49, 3509-3519.

### Synthesis of pyridine amines

The synthesis of (2-(4-methylpiperidin-1-yl)-6-(trifluoromethyl)pyridin-3-yl)methanamine **(PYR-1)** has been described in literature (WO2008125296, p. 56). The building blocks disclosed in Table 2 hereinbelow can be synthesized along the same route, using the respective amine.

**Table 2:**

| | |
|---|---|
| 3-(aminomethyl)-N-ethyl-6-(trifluoromethyl)pyridin-2-amine | **PYR-3** |
| (2-(pyrrolidin-1-yl)-6-(trifluoromethyl)pyridin-3-yl)methanamine | **PYR-4** |
| 3-(aminomethyl)-N-butyl-6-(trifluoromethyl)pyridin-2-amine | **PYR-5** |
| (2-morpholino-6-(trifluoromethyl)pyridin-3-yl)methanamine | **PYR-9** |
| (2-(piperidin-1-yl)-6-(trifluoromethyl)pyridin-3-yl)methanamine | **PYR-11** |

The synthesis of (2-cyclohexyl-6-(trifluoromethyl)pyridin-3-yl)methanamine has been described in Bioorg. Med. Chem. Lett. 2014, 24, 4039-4043. The pyridines disclosed in Table 3 hereinbelow can be synthesized along the same route, using the respective alkenyl boronic acids.

**Table 3:**

| | |
|---|---|
| (2-cyclopentyl-6-(trifluoromethyl)pyridin-3-yl)methanamine | **PYR-2** |
| (2-(tetrahydro-2H-pyran-4-yl)-6-(trifluoromethyl)pyridin-3-yl)methanamine | **PYR-8** |
| (2-(3-methoxypropyl)-6-(trifluoromethyl)pyridin-3-yl)methanamine | **PYR-10** |
| (2-(cyclohexyimethyl)-6-(trifiuoromethyl)pyridin-3-yl)methanamine | **PYR-13** |

The synthesis of (2-((tetrahydro-2H-pyran-4-yl)oxy)-6-(trifluoromethyl)pyridin-3-yl)methanamine **(PYR-6)** has been described US20130079373, pp. 20,21.

The synthesis of (2-(m-tolyl)-6-(trifluoromethyl)pyridin-3-yl)methanamine **(PYR-7)** has been described US20130079377, pp. 24,25.

### Synthesis of (2-((2-methoxyethoxy)methyl)-6-(trifluoromethyl)pyridin-3-yl)methanamine hydrochloride (PYR-12)

### Synthesis of ethyl 4-(2-methoxyethoxy)-3-oxobutanoate

To a stirred suspension of 60% sodium hydride (10.4g, 0.26 mol) in THF (100 mL) was added 2-methoxyethanol (21.56 g, 0.13 mol) at 10-15°C under N₂. The reaction mixture was stirred for 30 min. To this solution, ethyl 4-chloro-3-oxobutanoate (10 g, 0.13 mol) in THF (50 mL) was added and was allowed to stir for 2 h at RT. The reaction mixture was concentrated under reduced pressure, diluted with H₂O (150 mL) and was extracted with EE (3 x 100 mL). The combined organic layer was washed with brine (100 mL). The organic part was dried over anhydrous magnesium sulfate and was concentrated under reduced pressure to afford crude compound which was purified by SC (eluent: 15% EE in hexane) to afford ethyl 4-(2-methoxyethoxy)-3-oxobutanoate. Yield: 7.8 g (62%).

### Synthesis of (Z)-ethyl 3-amino-4-(2-methoxyethoxy)but-2-enoate

To a stirred solution of ethyl 4-(2-methoxyethoxy)-3-oxobutanoate (7.5 g, 36 mmol) in benzene (700 mL) was added ammonium acetate (14.16 g, 0.18 mol) and AcOH (3 mL). The reaction mixture was heated to reflux in dean stark apparatus for 12 h. The reaction mixture was concentrated after complete removal of H₂O under reduced pressure to afford crude (Z)-ethyl 3-amino-4-(2-methoxy-ethoxy)but-2-enoate. Yield: 10.2 g (crude).

### Synthesis of ethyl 2-((2-methoxyethoxy)methyl)-6-(trifluoromethyl)nicotinate

To a stirred solution of (Z)-ethyl 3-amino-4-(2-methoxyethoxy)but-2-enoate (10.2 g, 50 mmol) in acetonitrile (50 mL) was added (E)-4-ethoxy-1,1,1-trifluorobut-3-en-2-one (8.4 g, 50 mmol). The reaction mixture was allowed to stir for 30 min at RT and then reflux for 3h. The reaction mixture was concentrated under reduced pressure to afford crude compound which was purified by SC (eluent: 10% EE in hexane) to afford ethyl 2-((2-methoxyethoxy)methyl)-6-(trifluoromethyl)nicotinate. Yield: 5.1 g(33%).

### Synthesis of 2-((2-methoxyethoxy)methyl)-6-(trifluoromethyl)nicotinamide

To the stirred solution of ethyl 2-((2-methoxyethoxy)methyl)-6-(trifluoromethyl)nicotinate (5.1 g, 12.7 mmol) in EtOH (5 mL) was added ammonium hydroxide (50 mL). The reaction mixture was allowed to stir for 3 h at RT. The reaction mixture was concentrated under reduced pressure to get crude compound which was purified by SC (eluent: 10% EE in hexane) to afford 2-((2-methoxyethoxy)-methyl)-6-(trifluoromethyl)nicotinamide. Yield: 3.2 g (69.5%).

### Snyhthesis of tert-butyl ((2-((2-methoxyethoxy)methyl)-6-(trifluoromethyl)pyridin-3-yl)methyl)carbamate

To a stirred solution of sodium borohydride (0.65 g, 17.2 mmol) in THF (10 mL) was added BF₃-etharate (2.43 g, 17.2 mmol) under cooling and was allowed to stir for 30 min. To this suspension, 2-((2-methoxyethoxy)methyl)-6-(trifluoromethyl)nicotinamide (3.2 g, 11.5 mol) in THF (10 mL) was added and was allowed to stir for 12h at RT. The reaction mixture was cooled to 0°C, quenched with MeOH (15 mL) and was allowed stir for 1h. To this reaction mixture boc anhydride (3.74 g, 17.2 mmol) was added and was allowed to stir for 1 h. The reaction mixture was concentrated to dryness. The crude compound was purified through SC (eluent: 10% EE in hexane) to afford pure tert-butyl ((2-((2-methoxyethoxy)methyl)-6-(trifluoromethyl)pyridin-3-yl)methyl)carbamate. Yield: 1.7 g (41%).

### Synthesis of (2-((2-methoxyethoxy)methyl)-6-(trifluoromethyl)pyridin-3-yl)methanamine hydrochloride (PYR-12)

Tert-butyl ((2-((2-methoxyethoxy)methyl)-6-(trifluoromethyl)pyridin-3-yl)methyl)carbamate (1.7 g, 4.67 mmol) was dissolved in 2 mL of 1,4-dioxane and to the solution was added 1,4-dioxane in HCl (9 mL) and was allowed to stir at RT for 2 h. The reaction mixture was concentrated under reduced pressure and was co-distilled with MeOH twice to afford crude compound. The crude compound was filtered with 30% EE in hexane to afford pure (2-((2-methoxyethoxy)methyl)-6-(trifluoromethyl)pyridin-3-yl)methanamine hydrochloride **(PYR-12).** Yield: 1.18 g (98%).

### Synthesis of example compounds

The coupling of pyridine methylamines with carboxylic acids using 1-hydroxybenzotriazole and O(1H-benzotriazol-1-yl)-N,N,N',N'-tetramethyluronium tetrafluoroborate has been described in WO2013013817, p. 91. Accordingly, the example compounds disclosed in Table 4 hereinbelow have been prepared from the acids and pyridine methylamines described above.

**Table 4:**

| **comp. no.** | **chemical name** | **pyridine-amines** | **acids** |
|---|---|---|---|
| **1** | 2-(5-hydroxy-1-methyl-1H-indol-3-yl)-N-((2-(4-methylpiperidin-1-yl)-6-(trifluoromethyl)pyridin-3-yl)methyl)propanamide | **PYR-1** | **ACI-1** |
| **2** | 2-(5-methoxy-1-methyl-1H-indol-3-yl)-N-((2-(4-methylpiperidin-1-yl)-6-(trifluoromethyl)pyridin-3-yl)methyl)acetamide | **PYR-1** | **ACI-2** |
| **3** | N-((2-cyclopentyl-6-(trifluoromethyl)pyridin-3-yl)methyl)-2-(5-hydroxy-1-methyl-1H-indol-3-yl)propanamide | **PYR-2** | **ACI-1** |
| **4** | N-((2-(ethylamino)-6-(trifluoromethyl)pyridin-3-yl)methyl)-2-(5-hydroxy-1-methyl-1H-indol-3-yl)propanamide | **PYR-3** | **ACI-1** |
| **5** | 2-(5-hydroxy-1-methyl-1H-indol-3-yl)-N-((2-(pyrrolidin-1-yl)-6-(trifluoromethyl)pyridin-3-yl)methyl)propanamide | **PYR-4** | **ACI-1** |
| **6** | N-((2-(butylamino)-6-(trifluoromethyl)pyridin-3-yl)methyl)-2-(5-hydroxy-1-methyl-1H-indol-3-yl)propanamide | **PYR-5** | **ACI-1** |
| **7** | 2-(5-hydroxy-1-methyl-1H-indol-3-yl)-N-((2-((tetrahydro-2H-pyran-4-yl)oxy)-6-(trifluoromethyl)pyridin-3-yl)methyl)propanamide | **PYR-6** | **ACI-1** |
| **8** | 2-(5-hydroxy-1-methyl-1H-indol-3-yl)-N-((2-(m-tolyl)-6-(trifluoromethyl)pyridin-3-yl)methyl)propanamide | **PYR-7** | **ACI-1** |
| **9** | 2-(5-hydroxy-1-methyl-1H-indol-3-yl)-N-((2-(tetrahydro-2H-pyran-4-yl)-6-(trifluoromethyl)pyridin-3-yl)methyl)propanamide | **PYR-8** | **ACI-1** |
| **10** | 2-(5-hydroxy-1-methyl-1H-indol-3-yl)-N-((2-morpholino-6-(trifluoromethyl)pyridin-3-yl)methyl)propanamide | **PYR-9** | **ACI-1** |
| **11** | 2-(5-hydroxy-1-methyl-1H-indol-3-yl)-N-((2-(4-methylpiperidin-1-yl)-6-(trifluoromethyl)pyridin-3-yl)methyl)acetamide | **PYR-2** | **ACI-3** |
| **12** | 2-(5-hydroxy-1-methyl-1H-indol-3-yl)-N-((2-(3-methoxypropyl)-6-(trifluoromethyl)pyridin-3-yl)methyl)propanamide | **PYR-10** | **ACI-1** |
| **13** | 2-(5-hydroxy-1-methyl-1H-indol-3-yl)-N-((2-(piperidin-1-yl)-6-(trifluoromethyl)pyridin-3-yl)methyl)propanamide | **PYR-11** | **ACI-1** |
| **14** | 2-(1-methyl-1H-indol-3-yl)-N-((2-(4-methylpiperidin-1-yl)-6-(trifluoromethyl)pyridin-3-yl)methyl)propanamide | **PYR-1** | **ACI-4** |
| **15** | 2-(5-hydroxy-1-methyl-1H-indol-3-yl)-N-((2-((2-methoxyethoxy)methyl)-6-(trifluoromethyl)pyridin-3-yl)methyl)propanamide | **PYR-12** | **ACI-1** |
| **16** | N-((2-(ethylamino)-6-(trifluoromethyl)pyridin-3-yl)methyl)-2-(5-methoxy-1-methyl-1H-indol-3-yl)propanamide | **PYR-3** | **ACI-5** |
| **17** | N-((2-(ethylamino)-6-(trifluoromethyl)pyridin-3-yl)methyl)-2-(5-methoxy-1-methyl-1H-indol-3-yl)acetamide | **PYR-3** | **ACI-2** |
| **18** | N-((2-(ethylamino)-6-(trifluoromethyl)pyridin-3-yl)methyl)-2-(5-hydroxy-1-methyl-1H-indol-3-yl)acetamide | **PYR-3** | **ACI-3** |
| **19** | N-((2-(butylamino)-6-(trifluoromethyl)pyridin-3-yl)methyl)-2-(5-methoxy-1-methyl-1H-indol-3-yl)propanamide | **PYR-5** | **ACI-5** |
| **20** | 2-(5-hydroxy-1-methyl-1H-indol-3-yl)-N-((2-morpholino-6-(trifluoromethyl)pyridin-3-yl)methyl)acetamide | **PYR-9** | **ACI-3** |
| **21** | N-((2-cyclopentyl-6-(trifluoromethyl)pyridin-3-yl)methyl)-2-(5-hydroxy-1-methyl-1H-indol-3-yl)acetamide | **PYR-2** | **ACI-3** |
| **22** | N-((2-(cyclohexylmethyl)-6-(trifluoromethyl)pyridin-3-yl)methyl)-2-(1-methyl-1H-indol-3-yl)propanamide | **PYR-13** | **ACI-4** |

The conversion of hetarylamines to the corresponding phenylcarbamates and the subsequent coupling with pyridine methylamine to yield ureas has been described in WO2013013817, p. 94/95. Accordingly, the example compounds disclosed in Table 5 hereinbelow have been prepared from the hetarylamines and pyridine methylamines described above.

**Table 5:**

| **comp. no.** | **chemical name** | **pyridine-amines** | **amines** |
|---|---|---|---|
| **23** | 1-(4-chlorobenzo[d]thiazol-2-yl)-3-((2-(4-methylpiperidin-1-yl)-6-(trifluoromethyl)pyridin-3-yl)methyl)urea | **PYR-1** | **AMI-1** |
| **24** | 1-((2-(4-methylpiperidin-1-yl)-6-(trifluoromethyl)pyridin-3-yl)methyl)-3-(4-nitrobenzo[d]thiazol-2-yl)urea | **PYR-1** | **AMI-2** |
| **25** | 1-(benzo[d]thiazol-2-yl)-3-((2-(4-methylpipedin-1-yl)-6-(trifluoromethyl)pyridin-3-yl)methyl)urea | **PYR-1** | **AMI-3** |
| **26** | 1-(1H-benzo[d]imidazol-2-yl)-3-((2-(4-methylpiperidin-1-yl)-6-(trifluoromethyl)pyridin-3-yl)methyl)urea | **PYR-1** | **AMI-4** |

### Pharmacological methods

### Functional testing carried out on the vanilloid receptor 1 (VRI/TRPV1 receptor)

The agonistic or antagonistic effect of the substances to be tested on the rat-species vanilloid receptor 1 (VR1/TRPV1) can be determined using the following assay. In this assay, the influx of Ca²⁺ through the receptor channel is quantified with the aid of a Ca²⁺-sensitive dye (type Fluo-4, Molecular Probes Europe BV, Leiden, the Netherlands) in a fluorescent imaging plate reader (FLIPR, Molecular Devices, Sunnyvale, USA).

AA solution (antibiotic/antimyotic solution), type 2 collagenase and trypsin were obtained from PAA, Pasching, Austria. HAMS F12 nutrient mixture, FCS (foetal calf serum), NGF medium (2.5 S), laminin, PBS (Ca-Mg-free PBS) and HBSS buffer (Hank's buffered saline solution) were obtained from Gibco Invitrogen GmbH, Karlsruhe, Germany.

### Method:

Complete medium: 50 mL HAMS F12 nutrient mixture with 10 vol.-% of FCS (heat-inactivated); 2 mM L-glutamine (Sigma, Munich, Germany); 1 wt.-% of AA solution and 25 ng/mL NGF medium (2.5 S).

Cell culture plate: Poly-D-lysine-coated, black 96-well plates having a clear base (96-well black/clear plate, BD Biosciences, Heidelberg, Germany) are additionally coated with laminin, the laminin being diluted with PBS (Ca-Mg-free PBS) to a concentration of 100 µg/mL. Aliquots having a laminin concentration of 100 µg/mL are removed and stored at -20°C. The aliquots are diluted with PBS in a ratio of 1:10 to 10 µg/mL of laminin and respectively 50 µL of the solution are pipetted into a recess in the cell culture plate. The cell culture plates are incubated for at least two hours at 37°C, the excess solution is removed by suction and the recesses are each washed twice with PBS. The coated cell culture plates are stored with excess PBS which is not removed until just before the feeding of the cells.

### Preparation of the cells:

The vertebral column is removed from decapitated rats and placed immediately into cold HBSS buffer, i.e. buffer located in an ice bath, mixed with 1 vol.-% of an AA solution. The vertebral column is cut longitudinally and removed together with fasciae from the vertebral canal. Subsequently, the dorsal root ganglia (DRG) are removed and again stored in cold HBSS buffer mixed with 1 vol.-% of an AA solution. The DRG, from which all blood remnants and spinal nerves have been removed, are transferred in each case to 500 µL of cold type 2 collagenase and incubated for 35 minutes at 37°C. After the addition of 2.5 vol.-% of trypsin, incubation is continued for 10 minutes at 37°C. After complete incubation, the enzyme solution is carefully pipetted off and 500 µL of complete medium are added to each of the remaining DRG. The DRG are respectively suspended several times, drawn through cannulae No. 1, No. 12 and No. 16 using a syringe and transferred to a 50 mL Falcon tube which is filled up to 15 mL with complete medium. The contents of each Falcon tube are respectively filtered through a 70 µm Falcon filter element and centrifuged for 10 min at 1,200 rpm and RT. The resulting pellet is respectively taken up in 250 µL of complete medium and the cell count is determined.

The number of cells in the suspension is set to 3 x 10⁵ per mL and 150 µL of this suspension are in each case introduced into a recess in the cell culture plates coated as described hereinbefore. In the incubator the plates are left for two to three days at 37°C, 5 vol.-% of CO₂ and 95 % relative humidity. Subsequently, the cells are loaded with 2 µM of Fluo-4 and 0.01 vol.-% of Pluronic F127 (Molecular Probes Europe BV, Leiden, the Netherlands) in HBSS buffer for 30 min at 37°C, washed 3 times with HBSS buffer and after further incubation for 15 min at RT used for Ca²⁺ measurement in a FLIPR assay. The Ca²⁺-dependent fluorescence is in this case measured before and after the addition of substances (λex = 488 nm, λem = 540 nm). Quantification is carried out by measuring the highest fluorescence intensity (FC, fluorescence counts) over time.

### FLIPR assay:

The FLIPR protocol consists of 2 substance additions. First the compounds to be tested (10 µM) are pipetted onto the cells and the Ca²⁺ influx is compared with the control (capsaicin 10 µM). This provides the result in % activation based on the Ca²⁺ signal after the addition of 10 µM of capsaicin (CP). After 5 min incubation, 100 nM of capsaicin are applied and the Ca²⁺ influx is also determined. Desensitising agonists and antagonists lead to suppression of the Ca²⁺ influx. The % inhibition is calculated compared to the maximum achievable inhibition with 10 µM of capsazepine.

Triple analyses (n=3) are carried out and repeated in at least 3 independent experiments (N=4). Starting from the percentage displacement caused by different concentrations of the compounds to be tested of general formula I, IC₅₀ inhibitory concentrations which cause a 50% displacement of capsaicin were calculated.

Kᵢ values for the test substances were obtained by conversion by means of the Cheng-Prusoff equation (Cheng, Prusoff; Biochem. Pharmacol. 22, 3099-3108, 1973).

### Pharmacological data:

The affinity of selected compounds according to the invention for the vanilloid receptor 1 (VR1/TRPV1 receptor) was determined as described hereinbefore.

The compounds according to the invention display affinity to the VR1/TRPV1 receptor as shown in Table 6 given below. In said table pAG denotes partial agonist.

The value after the "@"symbol indicates the concentration at which the indicated inhibition (in %) was respectively determined.

The term "ne" denotes that the respective example compound did not show any effect in this assay.

**Table 6:**

| **example comp. no.** | **TRPV1 human (*f*) Ki [nM] CAP** | **example comp. no.** | **TRPV1 human *(f)* Ki [nM] CAP** | **example comp. no.** | **TRPV1 human *(f)* Ki [nM] CAP** |
|---|---|---|---|---|---|
| **1** | 0.8 | **10** | 29.3 | **19** | 76.4 |
| **2** | 29.6 | **11** | 0.3 | **20** | 16.5 |
| **3** | 4.8 | **12** | 81.0 | **21** | 8.0 |
| **4** | 44.2 | **13** | 3.9 | **22** | 35.9 |
| **5** | 5.0 | **14** | 53.0 | **23** | ne |
| **6** | 16.2 | **15** | 15% @ 5 µM | **24** | pAG (31.2) |
| **7** | 29.1 | **16** | ne | **25** | 84.9 |
| **8** | 1.4 | **17** | 35% @ 5 µM; 0% @ 1 µM | **26** | ne |
| **9** | 45.7 | **18** | 8.2 | | |

## Claims

1. A compound of formula (I) 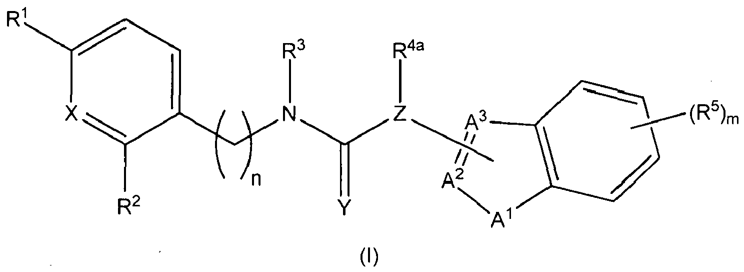 wherein
n represents 1, 2, 3 or 4;
m represents 0, 1, 2, 3 or 4;
X represents N or CH;
Y represents O, S, or N-CN;
Z represents N or CR^{4b};
A¹ represents S, 0 or NR⁶;
A² represents N or CR⁷;
A³ represents N or CR⁷;
with the proviso that one of R⁶ and R⁷ represents a bond to Z;
R¹ represents a C₁₋₄ aliphatic residue, a C₃₋₆ cycloaliphatic residue or a 3 to 6 membered heterocycloaliphatic residue, in each case unsubstituted or mono- or polysubstituted;
R² represents an unsubstituted or mono- or polysubstituted C₁₋₁₀ aliphatic residue,
a C₃₋₁₀-cycloaliphatic residue, a 3 to 10-membered heterocycloaliphatic residue, an aryl or a heteroaryl, in each case unsubstituted or mono- or polysubstituted and in each case optionally bridged via an unsubstituted or mono- or polysubstituted C₁₋₈ aliphatic group;
O-C₁₋₁₀ aliphatic residue, O-C₃₋₁₀ cycloaliphatic residue, 0-(3 to 10-membered heterocycloaliphatic residue), O-aryl or O-heteroaryl, in each case unsubstituted or mono-or polysubstituted;
(O-(CH₂)₁₋₃)₀₋₃-O-(CH₂)₀₋₃-CH₃, optionally bridged via an unsubstituted or mono- or polysubstituted C₁₋₈ aliphatic group;
S-C₁₋₁₀ aliphatic residue, S-C₃₋₁₀ cycloaliphatic residue, S-(3 to 10-membered heterocycloaliphatic residue), S-aryl or S-heteroaryl, in each case unsubstituted or mono-or polysubstituted;
NH₂;
N(H)-C₁₋₁₀ aliphatic residue, N(H)-C₃₋₁₀ cycloaliphatic residue, N(H)-(3 to 10-membered heterocycloaliphatic residue), N(H)-aryl or N(H)-heteroaryl, in each case unsubstituted or mono- or polysubstituted;
N(C₁₋₄ aliphatic residue)-C₁₋₁₀ aliphatic residue, N(C₁₋₄ aliphatic residue)-C₃₋₁₀ cycloaliphatic residue, N(C₁₋₄ aliphatic residue)-(3 to 10-membered heterocycloaliphatic residue), N(C₁₋₄ aliphatic residue)-aryl or N(C₁₋₄ aliphatic residue)-heteroaryl, in each case unsubstituted or mono- or polysubstituted;
R³ represents H or an unsubstituted or mono- or polysubstituted C₁₋₄ aliphatic residue;
R^{4a} represents H; a C₁₋₄ aliphatic residue, a C₃₋₆ cycloaliphatic residue or aryl, in each case unsubstituted or mono- or polysubstituted;
R^{4b} represents H; or an unsubstituted, mono- or polysubstituted C₁₋₄ aliphatic residue;
R⁵ each independently of one another represents F; Cl; Br; I; CN; CH₃; C₂₋₄-alkyl; CF₃; CF₂H; CFH₂; CF₂Cl; CFCl₂; C(=O)-H; C(=O)-(C₁₋₄-alkyl); C(=O)-OH;C(=O)-O-(C₁₋₄-alkyl); C(=O)-NH₂; C(=O)-N(H)(C₁₋₄-alkyl); C(=O)-N(C₁₋₄-alkyl)₂; OH; OCH₃; O-C₂₋₄-alkyl; OCF₃; OCF₂H; OCFH₂; OCF₂Cl; OCFCl₂; O-S(=O)₂-OH; O-S(=O)₂-O-C₁₋₄-alkyl; O-S(=O)₂-NH₂; O-S(=O)₂-N(H)(C₁₋₄-alkyl); O-S(=O)₂-N(C₁₋₄-alkyl)₂; NH₂; NO₂; N(H)(C₁₋₄-alkyl); N(C₁₋₄-alkyl)₂; NH-C(=O)-NH₂; NH-C(=O)-N(H)(C₁₋₄-alkyl); NH-C(=O)-N(C₁₋₄-alkyl)₂; NH-S(=O)₂-OH; NH-S(=O)₂-O-C₁₋₄-alkyl; NH-S(=O)₂-NH₂; NH-S(=O)₂-N(H)(C₁₋₄-alkyl); NH-S(=O)₂-N(C₁₋₄-alkyl)₂; SH; SCH₃; S-C₂₋₄-alkyl; SCF₃; SCF₂H; SCFH₂; SCF₂Cl; SCFCl₂; S(=O)₂-OH; S(=O)₂-O-C₁₋₄-alkyl; S(=O)₂-NH₂; S(=O)₂-N(H)(C₁₋₄-alkyl); or S(=O)₂-N(C₁₋₄-alkyl)₂;
R⁶ represents H; a C₁₋₄ aliphatic residue, a C₃₋₆-cycloaliphatic residue or a 3 to 6-membered heterocycloaliphatic residue, in each case unsubstituted or mono- or polysubstituted;
R⁷ represents bond; H; F; Cl; Br; I; CN; CH₃; C₂₋₄-alkyl; CF₃; CF₂H; CFH₂; CF₂Cl; CFCl₂; C(=O)-H; C(=O)-(C₁₋₄-alkyl); C(=O)-OH;C(=O)-O-(C₁₋₄-alkyl); C(=O)-NH₂; C(=O)-N(H)(C₁₋₄-alkyl); C(=O)-N(C₁₋₄-alkyl)₂; OH; OCH₃; O-C₂₋₄-alkyl; OCF₃; OCF₂H; OCFH₂; OCF₂Cl; OCFCl₂; O-S(=O)₂-OH; O-S(=O)₂-O-C₁₋₄-alkyl; O-S(=O)₂-NH₂; O-S(=O)₂-N(H)(C₁₋₄-alkyl); O-S(=O)₂-N(C₁₋₄-alkyl)₂; NH₂; NO₂; N(H)(C₁₋₄-alkyl); N(C₁₋₄-alkyl)₂; NH-C(=O)-NH₂; NH-C(=O)-N(H)(C₁₋₄-alkyl); NH-C(=O)-N(C₁₋₄-alkyl)₂; NH-S(=O)₂-OH; NH-S(=O)₂-O-C₁₋₄-alkyl; NH-S(=O)₂-NH₂; NH-S(=O)₂-N(H)(C₁₋₄-alkyl); NH-S(=O)₂-N(C₁₋₄-alkyl)₂; SH; SCH₃; S-C₂₋₄-alkyl; SCF₃; SCF₂H; SCFH₂; SCF₂Cl; SCFCl₂; S(=O)₂-OH; S(=O)₂-O-C₁₋₄-alkyl; S(=O)₂-NH₂; S(=O)₂-N(H)(C₁₋₄-alkyl); or S(=O)₂-N(C₁₋₄-alkyl)₂;
in which an "aliphatic group" and an "aliphatic residue" can in each case, independently of one another, be branched or unbranched, saturated or unsaturated;
in which a "cycloaliphatic residue" and a "heterocycloaliphatic residue" can in each case, independently of one another, be saturated or unsaturated;
in which "mono- or polysubstituted" with respect to an "aliphatic group", an "aliphatic residue", a "cycloaliphatic residue" and a "heterocycloaliphatic residue" relates in each case independently of one another, with respect to the corresponding residues or groups, to the substitution of one or more hydrogen atoms each independently of one another by at least one substituent selected from the group consisting of F; Cl; Br; I; CN; CH₃; C₂₋₄-alkyl; CF₃; CF₂H; CFH₂; CF₂Cl; CFCl₂; C(=O)-H; C(=O)-(C₁₋₄-alkyl); C(=O)-OH;C(=O)-O-(C₁₋₄-alkyl); C(=O)-NH₂; C(=O)-N(H)(C₁₋₄-alkyl); C(=O)-N(C₁₋₄-alkyl)₂; OH; OCH₃; O-C₂₋₄-alkyl; OCF₃; OCF₂H; OCFH₂; OCF₂Cl; OCFCl₂; 0-S(=O)₂-OH; O-S(=O)₂-O-C₁₋₄-alkyl; O-S(=O)₂-NH₂; O-S(=O)₂-N(H)(C₁₋₄-alkyl); O-S(=O)₂-N(C₁₋₄-alkyl)₂; NH₂; NO₂; N(H)(C₁₋₄-alkyl); N(C₁₋₄-alkyl)₂; NH-C(=O)-NH₂; NH-C(=O)-N(H)(C₁₋₄-alkyl); NH-C(=O)-N(C₁₋₄-alkyl)₂; NH-S(=O)₂-OH; NH-S(=O)₂-O-C₁₋₄-alkyl; NH-S(=O)₂-NH₂; NH-S(=O)₂-N(H)(C₁₋₄-alkyl); NH-S(=O)₂-N(C₁₋₄-alkyl)₂; SH; SCH₃; S-C₂₋₄-alkyl; SCF₃; SCF₂H; SCFH₂; SCF₂Cl; SCFCl₂; S(=O)₂-OH; S(=O)₂-O-C₁₋₄-alkyl: S(=O)₂-NH₂; S(=O)₂-N(H)(C₁₋₄-alkyl); or S(=O)₂-N(C₁₋₄-alkyl)₂;
in which "mono- or polysubstituted" with respect to "aryl" and a "heteroaryl" relates, with respect to the corresponding residues, in each case independently of one another, to the substitution of one or more hydrogen atoms each independently of one another by at least one substituent selected from the group consisting of F; Cl; Br; I; CN; CH₃; C₂₋₄-alkyl; CF₃; CF₂H; CFH₂; CF₂Cl; CFCl₂; C(=O)-H; C(=O)-(C₁₋₄-alkyl); C(=O)-OH;C(=O)-O-(C₁₋₄-alkyl); C(=O)-NH₂; C(=O)-N(H)(C₁₋₄-alkyl); C(=O)-N(C₁₋₄-alkyl)₂; OH; OCH₃; O-C₂₋₄-alkyl; OCF₃; OCF₂H; OCFH₂; OCF₂Cl; OCFCl₂: O-S(=O)₂-OH; O-S(=O)₂-O-C₁₋₄-alkyl; O-S(=O)₂-NH₂; O-S(=O)₂-N(H)(C₁₋₄-alkyl); O-S(=O)₂-N(C₁₋₄-alkyl)₂; NH₂; NO₂; N(H)(C₁₋₄-alkyl); N(C₁₋₄-alkyl)₂; NH-C(=O)-NH₂; NH-C(=O)-N(H)(C₁₋₄-alkyl); NH-C(=O)-N(C₁₋₄-alkyl)₂; NH-S(=O)₂-OH; NH-S(=O)₂-O-C₁₋₄-alkyl; NH-S(=O)₂-NH₂; NH-S(=O)₂-N(H)(C₁₋₄-alkyl); NH-S(=O)₂-N(C₁₋₄-alkyl)₂; SH; SCH₃; S-C₂₋₄-alkyl; SCF₃; SCF₂H; SCFH₂; SCF₂Cl; SCFCl₂; S(=O)₂-OH; S(=O)₂-O-C₁₋₄-alkyl; S(=O)₂-NH₂; S(=O)₂-N(H)(C₁₋₄-alkyl); or S(=O)₂-N(C₁₋₄-alkyl)₂;
optionally in the form of a single stereoisomer or a mixture of stereoisomers, in the form of the free compound and/or a physiologically acceptable salt thereof.

2. The compound according to claim 1, **characterized in that** n represents 1.

3. The compound according to claim 1 or 2, **characterized in that** Y represents O.

4. The compound according to any of the preceding claims, **characterized in that**
R¹ is selected from the group consisting of CF₃, CH₃, CH₂CH₃, (CH₂)₂CH₃, CH(CH₃)₂, (CH₂)₃CH₃, CH(CH₃)CH₂CH₃, and C(CH₃)₃, or
is selected from the group consisting of cyclopropyl, cyclobutyl, cyclopentyl, and cyclohexyl.

5. The compound according to any of the preceding claims, **characterized in that**
R² represents substructure (T) in which 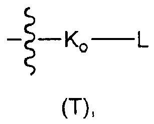
K represents O, an unsubstituted C₁₋₆ aliphatic group or NR⁸,
wherein R⁸ represents H or a C₁₋₄ aliphatic residue, unsubstituted or mono- or polysubstituted with one or more substituents each selected independently of one another from the group consisting of F, Cl, Br, I and C₁₋₄ alkyl;
o represents 0 or 1;
L represents an unsubstituted or mono- or polysubstituted C₃₋₆-cycloaliphatic residue,
an unsubstituted or mono- or polysubstituted 3 to 6-membered heterocycloaliphatic residue containing 1 to 3 heteroatoms, wherein binding to K occurs via a heteroatom or a carbon atom,
an unsubstituted or mono- or polysubstituted aryl,
an unsubstituted or mono- or polysubstituted heteroaryl containing 1 to 3 heteroatoms, wherein binding to K occurs via a heteroatom or a carbon atom, (O-(CH₂)₁₋₃)₀₋₃-O-(CH₂)₀₋₃-CH₃,
N(H)-C₁₋₆ aliphatic residue, unsubstituted or mono- or polysubstituted,
in which "mono- or polysubstituted" with respect to the aforementioned aliphatic residue, cycloaliphatic residue, heterocycloaliphatic residue, aryl and heteroaryl relates in each case independently of one another, with respect to the corresponding residues, to the substitution of one or more hydrogen atoms each independently of one another by at least one substituent selected from the group consisting of F; Cl; Br; I; CN; CH₃; C₂₋₄-alkyl; CF₃; OH; OCH₃; O-C₂₋₄-alkyl; OCF₃; OCF₂H; OCFH₂; NO₂; N(H)(C₁₋₄-alkyl); N(C₁₋₄-alkyl)₂.

6. The compound according to any of the preceding claims, **characterized in that** R³ is selected from the group consisting of H, CH₃ and CH₂CH₃.

7. The compound according to any of the preceding claims, **characterized in that**
R^{4a} represents H, CH₃, CH₂CH₃, cyclopropyl, cyclobutyl, cyclopentyl, cyclohexyl, or phenyl, wherein phenyl is unsubstituted or substituted with 1, 2, 3, 4 or 5 substituents independently selected from the group consisting of F, Cl, Br, I, NO₂, CN, CF₃, CF₂H, CFH₂, CF₂Cl, CFCl₂, OH, NH₂, NH(C₁₋₄ alkyl) and N(C₁₋₄ alkyl)(C₁₋₄ alkyl), C₁₋₄ alkyl, and 0-C₁₋₄-alkyl; and
R^{4b} represents H, CH₃ or CH₂CH₃.

8. The compound according to any of the preceding claims, **characterized in that** the partial structure (E) 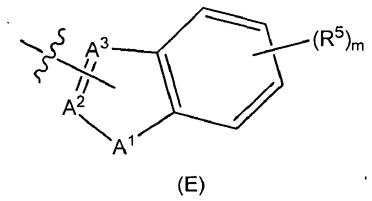 represents a moiety selected from the group consisting of: 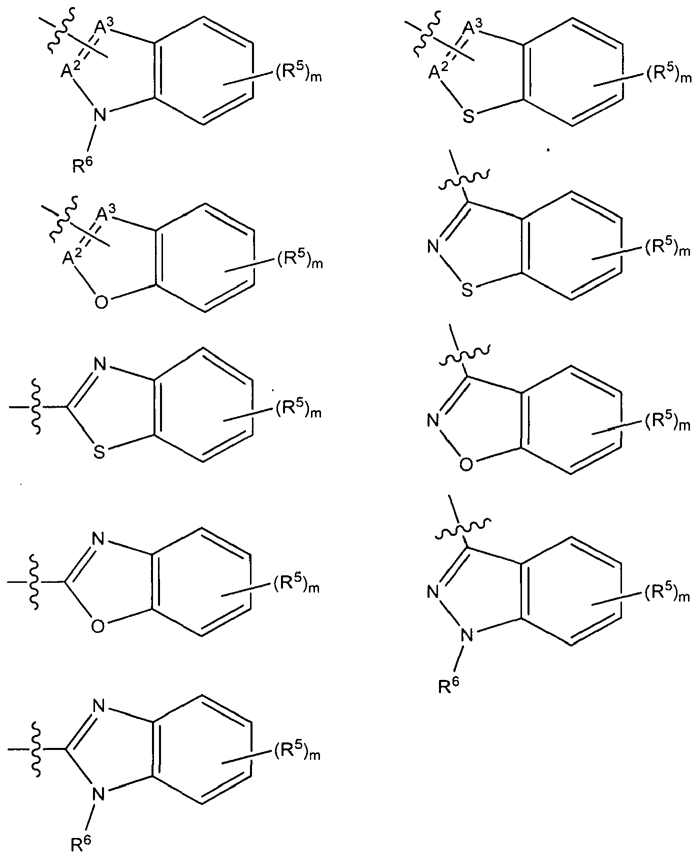 wherein
A² and A³ represent CR⁷ with R⁷ representing bond, H, CH₃ or C₂₋₄-alkyl.

9. The compound according to any of the preceding claims, **characterized in that** m is 0 or 1 and
R⁵ represents F; Cl; Br; I; CN; CH₃; CF₃; OH; OCH₃; O-C₂₋₄-alkyl; OCF₃; NO₂.

10. The compound according to any of the preceding claims, **characterized in that**
n represents 1;
Y represents O;
R¹ represents CF₃, CH₃ or cyclopropyl;
X represents N;
R³ represents H;
Z represents N or CR^{4b}, wherein R^{4b} represents H or CH₃;
R^{4a} represents H or CH₃;
R² represents substructure (T) in which 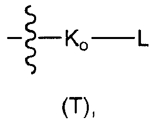
K represents O or an unsubstituted C₁₋₃ aliphatic group;
o represents 0 or 1;
L represents an unsubstituted or monosubstituted C₅₋₆-cycloaliphatic residue,
an unsubstituted or monosubstituted 5 to 6-membered heterocycloaliphatic residue containing 1 or 2 heteroatoms, wherein binding to K occurs via a heteroatom or a carbon atom,
an unsubstituted or monosubstituted 5 to 6-membered aryl,
an unsubstituted or monosubstituted 5 to 6-membered heteroaryl containing 1 to 3 heteroatoms, wherein binding to K occurs via a heteroatom or a carbon atom, (O-(CH₂)₁₋₃)₀₋₃-O-(CH₂)₀₋₃-CH₃,
NH-C₁₋₆ unsubstituted aliphatic residue,
in which "monosubstituted" with respect to the aforementioned residues relates in each case independently of one another, with respect to the corresponding residues, to the substitution of one hydrogen atom by one substituent selected from the group consisting of CH₃, CH₂CH₃, (CH₂)₂CH₃, CH(CH₃)₂, (CH₂)₃CH₃, CH(CH₃)CH₂CH₃, and C(CH₃)₃ and CF₃;
partial structure (E) is selected from the group consisting of 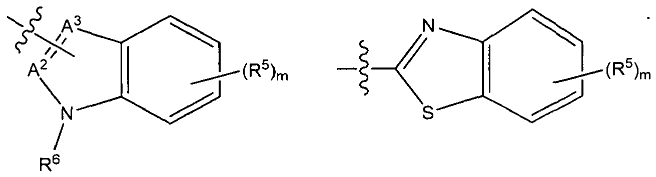 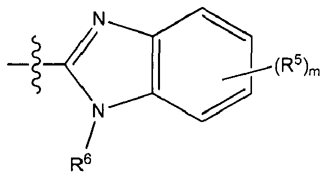 wherein
A² and A³ represent CR⁷ with R⁷ representing bond or H;
m represents 0 or 1, and
R⁵ represents OH; OCH₃; NO₂; F; Cl; Br or I.

11. The compound according to any of the preceding claims, selected from the group consisting of
**1** 2-(5-hydroxy-1-methyl-1H-indol-3-yl)-N-((2-(4-methylpiperidin-1-yl)-6-(trifluoromethyl)pyridin-3-yl)methyl)propanamide
**2** 2-(5-methoxy-1-methyl-1H-indol-3-yl)-N-((2-(4-methylpiperidin-1-yl)-6-(trifluoromethyl)pyridin-3-yl)methyl)acetamide
**3** N-((2-cyclopentyl-6-(trifluoromethyl)pyridin-3-yl)methyl)-2-(5-hydroxy-1-methyl-1H-indol-3-yl)propanamide
**4** N-((2-(ethylamino)-6-(trifluoromethyl)pyridin-3-yl)methyl)-2-(5-hydroxy-1-methyl-1H-indol-3-yl)propanamide
**5** 2-(5-hydroxy-1-methyl-1H-indol-3-yl)-N-((2-(pyrrolidin-1-yl)-6-(trifluoromethyl)pyridin-3-yl)methyl)propanamide
**6** N-((2-(butylamino)-6-(trifluoromethyl)pyridin-3-yl)methyl)-2-(5-hydroxy-1-methyl-1H-indol-3-yl)propanamide
**7** 2-(5-hydroxy-1-methyl-1H-indol-3-yl)-N-((2-((tetrahydro-2H-pyran-4-yl)oxy)-6-(trifluoromethyl)pyridin-3-yl)methyl)propanamide
**8** 2-(5-hydroxy-1-methyl-1H-indol-3-yl)-N-((2-(m-tolyl)-6-(trifluoromethyl)pyridin-3-yl)methyl)propanamide
**9** 2-(5-hydroxy-1-methyl-1H-indol-3-yl)-N-((2-(tetrahydro-2H-pyran-4-yl)-6-(trifluoromethyl)pyridin-3-yl)methyl)propanamide
**10** 2-(5-hydroxy-1-methyl-1H-indol-3-yl)-N-((2-morpholino-6-(trifluoromethyl)pyridin-3-yl)methyl)propanamide
**11** 2-(5-hydroxy-1-methyl-1H-indol-3-yl)-N-((2-(4-methylpiperidin-1-yl)-6-(trifluoromethyl)pyridin-3-yl)methyl)acetamide
**12** 2-(5-hydroxy-1-methyl-1H-indol-3-yl)-N-((2-(3-methoxypropyl)-6-(trifluoromethyl)pyridin-3-yl)methyl)propanamide
**13** 2-(5-hydroxy-1-methyl-1H-indol-3-yl)-N-((2-(piperidin-1-yl)-6-(trifluoromethyl)pyridin-3-yl)methyl)propanamide
**14** 2-(1-methyl-1H-indol-3-yl)-N-((2-(4-methylpiperidin-1-yl)-6-(trifluoromethyl)pyridin-3-yl)methyl)propanamide
**15** 2-(5-hydroxy-1-methyl-1 H-indol-3-yl)-N-((2-((2-methoxyethoxy)methyl)-6-(trifluoromethyl)pyridin-3-yl)methyl)propanamide
**16** N-((2-(ethylamino)-6-(trifluoromethyl)pyridin-3-yl)methyl)-2-(5-methoxy-1-methyl-1H-indol-3-yl)propanamide
**17** N-((2-(ethylamino)-6-(trifluoromethyl)pyridin-3-yl)methyl)-2-(5-methoxy-1-methyl-1H-indol-3-yl)acetamide
**18** N-((2-(ethylamino)-6-(trifluoromethyl)pyridin-3-yl)methyl)-2-(5-hydroxy-1-methyl-1H-indol-3-yl)acetamide
**19** N-((2-(butylamino)-6-(trifluoromethyl)pyridin-3-yl)methyl)-2-(5-methoxy-1-methyl-1H-indol-3-yl)propanamide
**20** 2-(5-hydroxy-1-methyl-1H-indol-3-yl)-N-((2-morpholino-6-(trifluoromethyl)pyridin-3-yl)methyl)acetamide
**21** N-((2-cyclopentyl-6-(trifluoromethyl)pyridin-3-yl)methyl)-2-(5-hydroxy-1-methyl-1H-indol-3-yl)acetamide
**22** N-((2-(cyclohexylmethyl)-6-(trifluoromethyl)pyridin-3-yl)methyl)-2-(1-methyl-1H-indol-3-yl)propanamide
**23** 1-(4-chlorobenzo[d]thiazol-2-yl)-3-((2-(4-methylpiperidin-1-yl)-6-(trifluoromethyl)pyridin-3-yl)methyl)urea
**24** 1-((2-(4-methylpiperidin-1 -yl)-6-(trifl uo rom ethyl) py rid in -3-yl) meth yl)-3 -(4-nitrobenzo[d]thiazol-2-yl)urea
**25** 1-(benzo[d]thiazol-2-yl)-3-((2-(4-methylpiperidin-1-yl)-6-(trifluoromethyl)pyridin-3-yl)methyl)urea
**26** 1-(H-benzo[d]imidazol-2-yl)-3-((2-(4-methylpiperidin-1-yl)-6-(trifluoromethyl)pyridin-3-yl)methyl)urea
optionally in the form of a single stereoisomer or a mixture of stereoisomers, in the form of the free compound and/or a physiologically acceptable salt thereof.

12. A pharmaceutical composition comprising at least one compound according to any of claims 1 to 11.

13. A compound according to any of claims 1 to 11 for use in the treatment and/or prophylaxis of one or more diseases and/or disorders selected from the group consisting of pain, preferably of pain selected from the group consisting of acute pain, chronic pain, neuropathic pain, visceral pain and joint pain; hyperalgesia; allodynia; causalgia; migraine; depression; nervous affection; axonal injuries; neurodegenerative diseases, preferably selected from the group consisting of multiple sclerosis, Alzheimer's disease, Parkinson's disease and Huntington's disease; cognitive dysfunctions, preferably cognitive deficiency states, particularly preferably memory disorders; epilepsy; respiratory diseases, preferably selected from the group consisting of asthma, bronchitis and pulmonary inflammation; coughs; urinary incontinence; overactive bladder (OAB); disorders and/or injuries of the gastrointestinal tract; duodenal ulcers; gastric ulcers; irritable bowel syndrome; strokes; eye irritations; skin irritations; neurotic skin diseases; allergic skin diseases; psoriasis; vitiligo; herpes simplex; inflammations, preferably inflammations of the intestine, the eyes, the bladder, the skin or the nasal mucous membrane; diarrhoea; pruritus; osteoporosis; arthritis; osteoarthritis; rheumatic diseases; eating disorders, preferably selected from the group consisting of bulimia, cachexia, anorexia and obesity; medication dependency; misuse of medication; withdrawal symptoms in medication dependency; development of tolerance to medication, preferably to natural or synthetic opioids; drug dependency; misuse of drugs; withdrawal symptoms in drug dependency; alcohol dependency; misuse of alcohol and withdrawal symptoms in alcohol dependency; for diuresis; for antinatriuresis; for influencing the cardiovascular system; for increasing vigilance; for the treatment of wounds and/or burns; for the treatment of severed nerves; for increasing libido; for modulating movement activity; for anxiolysis; for local anaesthesia and/or for inhibiting undesirable side effects, preferably selected from the group consisting of hyperthermia, hypertension and bronchoconstriction, triggered by the administration of vanilloid receptor 1 (VR1/TRPV1 receptor) agonists, preferably selected from the group consisting of capsaicin, resiniferatoxin, olvanil, arvanil, SDZ-249665, SDZ-249482, nuvanil and capsavanil.

14. A method of treatment and/or prophylaxis of disorders and/or diseases selected from the group consisting of pain, preferably of pain selected from the group consisting of acute pain, chronic pain, neuropathic pain, visceral pain and joint pain; hyperalgesia; allodynia; causalgia; migraine; depression; nervous affection; axonal injuries; neurodegenerative diseases, preferably selected from the group consisting of multiple sclerosis, Alzheimer's disease, Parkinson's disease and Huntington's disease; cognitive dysfunctions, preferably cognitive deficiency states, particularly preferably memory disorders; epilepsy; respiratory diseases, preferably selected from the group consisting of asthma, bronchitis and pulmonary inflammation; coughs; urinary incontinence; overactive bladder (OAB); disorders and/or injuries of the gastrointestinal tract; duodenal ulcers; gastric ulcers; irritable bowel syndrome; strokes; eye irritations; skin irritations; neurotic skin diseases; allergic skin diseases; psoriasis; vitiligo; herpes simplex; inflammations, preferably inflammations of the intestine, the eyes, the bladder, the skin or the nasal mucous membrane; diarrhoea; pruritus; osteoporosis; arthritis; osteoarthritis; rheumatic diseases; eating disorders, preferably selected from the group consisting of bulimia, cachexia, anorexia and obesity; medication dependency; misuse of medication; withdrawal symptoms in medication dependency; development of tolerance to medication, preferably to natural or synthetic opioids; drug dependency; misuse of drugs; withdrawal symptoms in drug dependency; alcohol dependency; misuse of alcohol and withdrawal symptoms in alcohol dependency; for diuresis; for antinatriuresis; for influencing the cardiovascular system; for increasing vigilance; for the treatment of wounds and/or burns; for the treatment of severed nerves; for increasing libido; for modulating movement activity; for anxiolysis; for local anaesthesia and/or for inhibiting undesirable side effects, preferably selected from the group consisting of hyperthermia, hypertension and bronchoconstriction, triggered by the administration of vanilloid receptor 1 (VR1/TRPV1 receptor) agonists, preferably selected from the group consisting of capsaicin, resiniferatoxin, olvanil, arvanil, SDZ-249665, SDZ-249482, nuvanil and capsavanil, which comprises administering an effective amount of at least one compound according to any of claims 1 to 11 to the mammal.
